# EUROPEAN PATENT APPLICATION

(11) **EP 2 502 674 A1**
(43) Date of publication of application: **26.09.2012**
(21) Application number: 11159123.6
(22) Date of filing: 22.03.2011
(51) Int. Cl.: B01L 3/00, C12Q 1/68, B01J 19/00, G01N 35/08

(54) **Method for performing molecular reactions by using immiscible intermediate fluids**

(71) Applicant: Koninklijke Philips Electronics N.V., 5656 AE Eindhoven (NL)
(72) Inventor: The designation of the inventor has not yet been filed
(74) Representative: Jungen, Carmen

(57) **Abstract**

The present invention relates to a method for performing molecular reactions in a device comprising the steps of (a) introducing one or more reagent solution(s) and an immiscible intermediate fluid into the device, wherein the device comprises a substrate, on which chemically or biochemically recognizable entities are immobilized; (b) performing molecular reactions between the immobilized chemically or biochemically recognizable entities and the reagent solution(s); or on the immobilized chemically or biochemically recognizable entities in the presence of the reagent solution(s); (c) displacing the reagent solution(s) present on the substrate by the immiscible intermediate fluid; (d) separating the immiscible intermediate fluid and the reagent solution(s); and reusing the reagent solution(s) and/or immiscible intermediate fluid for one or more repetitions of steps (a) to (e). The invention further relates to a device for performing a molecular reaction, comprising a reaction zone, reservoirs and liquid connections and a collection and regeneration zone wherein the immiscible intermediate fluid and the reagent solution(s) are separable by gravitational separation; or a redirection and distribution module, wherein the immiscible intermediate fluid and reagent solution(s) are separated. The invention also relates to the use of an immiscible intermediate fluid for displacing a reagent solution present in a reaction zone in a microfluidic device, as well as the use of a corresponding device for performing a sequencing reaction or a nucleic acid synthesis reaction.

## Description

### FIELD OF THE INVENTION

The present invention relates to a method for performing molecular reactions in a device comprising the steps of (a) introducing one or more reagent solution(s) and an immiscible intermediate fluid into the device, wherein the device comprises a substrate, on which chemically or biochemically recognizable entities are immobilized; (b) performing molecular reactions between the immobilized chemically or biochemically recognizable entities and the reagent solution(s); or on the immobilized chemically or biochemically recognizable entities in the presence of the reagent solution(s); (c) displacing the reagent solution(s) present on the substrate by the immiscible intermediate fluid; (d) separating the immiscible intermediate fluid and the reagent solution(s); and reusing the reagent solution(s) and/or immiscible intermediate fluid for one or more repetitions of steps (a) to (e). The invention further relates to a device for performing a molecular reaction, comprising a reaction zone, reservoirs and liquid connections and a collection and regeneration zone wherein the immiscible intermediate fluid and the reagent solution(s) are separable by gravitational separation; or a redirection and distribution module, wherein the immiscible intermediate fluid and reagent solution(s) are separated. The invention also relates to the use of an immiscible intermediate fluid for displacing a reagent solution present in a reaction zone in a microfluidic device, as well as the use of a corresponding device for performing a sequencing reaction or a nucleic acid synthesis reaction.

### BACKGROUND OF THE INVENTION

Modem molecular reaction devices are frequently based upon micromanipulation of discrete fluid droplets or unit-sized packets of fluids. These packets can be transported, stored, mixed, reacted, and/or analyzed in a discrete manner using a set of basic instructions. In order to establish the package nature of the fluids and to prevent a coalescence of adjacent droplets, immiscible carrier fluids can be used (Chen et al., 2007, Langmuir, 23, 2255-2260). Such immiscible carrier fluids are typically stable fluorocarbon-based fluids with high boiling points, high density, a high viscosity and a significantly reduced solubility of water. According to Chen et al, 2007 fluorocarbon-based fluids including FC-3283 and FC-40 can be used as spacer liquids for protein crystallization reactions in separated reagent plugs. However, the implementation of this approach has been shown only for a small number of cases including the synthesis of microparticles (Xu et al., 2005, Angew. Chem. Int. Ed., 44, 724-728) or the double emulsions (Utada et al., 2005, Science, 308, 537-541).

In other molecular technology sectors, e.g. in the field of DNA sequencing, current approaches are based on highly parallel molecular reactions in large arrays with relatively short readings lengths. This is due to the fact that the addition of a base to a DNA strand takes about less than 1 hour per base pair. Thus, the addition of around 100 base pairs requires about 3.5 to 4 days of run time, forming a substantial fraction of the typical 1 week sequencing run time in current sequencing machines. One of the reasons for the low speed of sequencing is the limited reaction rate for nucleotide incorporation, which is restricted by the concentration of enzymes or other reactants used in the reaction mixture for cost reasons. More importantly, since after every step of nucleotide incorporation the entire surface needs to be washed, all reagents are usually wasted. The total reagent consumption is therefore largely proportional to the read length and constitutes a main cost factor. This, in turn, leads to the tendency to decrease the reagent concentrations as much as possible with the downside of longer reaction times.

There is thus a need for a new methodology, which allows performing molecular reactions in a device without wasting reagent solutions, and concomitantly contributes to an overall cost reduction and an increase of the reaction speed.

### SUMMARY OF THE INVENTION

The present invention addresses this need and provides means and methods, which allow for performing molecular reactions with reduced overall costs and a potentially increased speed. The objective is, in particular, accomplished by provisions for a reuse of reagent solutions, more concretely by a method for performing molecular reactions in a device comprising the steps:
(a) introducing one or more reagent solution(s) and an immiscible intermediate fluid into the device, wherein the device comprises a substrate, on which chemically or biochemically recognizable entities are immobilized;
(b) performing molecular reactions between the immobilized chemically or biochemically recognizable entities and the reagent solution(s); or on the immobilized chemically or biochemically recognizable entities in the presence of the reagent solution(s);
(c) displacing the reagent solution(s) present on the substrate by the immiscible intermediate fluid;
(d) separating the immiscible intermediate fluid and the reagent solution(s), preferably in a collection and regeneration zone; and
(e) reusing the reagent solution(s) and/or immiscible intermediate fluid for one or more repetitions of steps (a) to (e).

This method provides several advantages: the employment of immiscible intermediate fluids permits the prevention of contamination and/or of dilution of reagents typically used in molecular reactions and, in consequence, paves the way for an effective reuse of these reagents. Furthermore, due to the possibility of reusing the reagents, an increase of the concentrations of reactants, in particular of rate limiting chemicals such as enzymes or labeled nucleotides, becomes possible. This, in turn can speed up the reactions in certain situations an order of magnitude while still benefiting from reduced overall reagent costs due to repeated recycling actions. In the ambit of sequencing reactions this means that the sequencing reactions run much cheaper and faster and enable the whole sequencing process, including sample prep and bio-informatics analysis, to be done within a day. Similar improvements are possible in other molecular reaction situations, e.g. in nucleic acid or protein synthesis approaches.

In a further preferred embodiment of the present invention the above described method additionally comprises the step of washing said substrate with a washing buffer, wherein said washing buffer is detached from said reagent solution(s) by the immiscible intermediate fluid.

In another preferred embodiment the herein above described methods additionally comprise the step of measuring the state or outcome of said molecular reaction. In particularly preferred embodiment said measuring may be carried out by optical and/or electrical means.

In another preferred embodiment of the present invention said displacing step (c) of the herein above described methods is to be performed by pneumatic fluid driving, peristaltic actuation, piston and actuated membrane driving or the application of vectored vacuum.

In another preferred embodiment of the present invention said separating step (d) of the herein above described methods is to be performed by allowing said reagent solution(s) and said immiscible intermediate fluid to separate by gravitational separation.

In another aspect the present invention relates to a device for performing a molecular reaction, comprising:
(a) a reaction zone which comprises a substrate, on which chemically or biochemically recognizable entities are immobilized;
(b) reservoirs and liquid connections allowing a reagent solution, an immiscible intermediate fluid and optionally a washing buffer to pass through the reaction zone, wherein said immiscible intermediate fluid is capable of displacing said reagent solution from said reaction zone; and
(c) a collection and regeneration zone wherein the immiscible intermediate fluid and the reagent solution are separable by gravitational separation, allowing for a reuse of the immiscible intermediate fluid and the reagent solution by channeling back the separated fluids to the reaction zone. In a particularly preferred embodiment said collection and regeneration zone comprises one inlet connected with the reaction zone and, in a further preferred embodiment, additionally comprises two outlets connected with a reservoir for the reagent solution and a reservoir for the immiscible intermediate fluid, respectively.

In another aspect the present invention relates to a device for performing a molecular reaction, comprising
(a) a reaction zone which comprises a substrate, on which chemically or biochemically recognizable entities are immobilized;
(b) reservoirs and liquid connections allowing a reagent solution, an immiscible intermediate fluid and optionally a washing buffer to pass through the reaction zone, wherein said immiscible intermediate fluid is capable of displacing said reagent solution from said reaction zone; and
(c) a redirection and distribution module, wherein the transition from the immiscible intermediate fluid to the reagent solution(s) is detected and said fluid and solution(s) are separated, allowing for a reuse of the immiscible intermediate fluid and the reagent solution by channeling back the separated fluids to the reaction zone. In a particularly preferred embodiment said redirection and distribution module comprises one inlet connected with the reaction zone and, in a further preferred embodiment, additionally comprises two outlets connected with a reservoir for the reagent solution and a reservoir for the immiscible intermediate fluid, respectively.

In a preferred embodiment of the present invention the herein above mentioned device is a microfluidic device. In a particularly preferred embodiment said device is an integrated microfluidic cartridge.

In a further preferred embodiment said herein above mentioned molecular reaction is a sequencing reaction or a nucleic acid synthesis reaction.

In another aspect the present invention relates to the use of an immiscible intermediate fluid for displacing a reagent solution present in a reaction zone in a microfluidic device from said reaction zone substrate, wherein the reaction zone comprises a substrate with chemically or biochemically recognizable entities and rendering said chemically of biochemically recognizable units (re-)active.

In a preferred embodiment of the present invention said herein above mentioned chemically or biochemically recognizable entities are nucleic acids, peptides, polypeptides, carbohydrates, modified versions thereof, an array of nucleic acids, peptides, polypeptides, carbohydrates or modified versions thereof or any combination thereof.

In yet another embodiment of the present invention said herein above mentioned immiscible intermediate fluid comprises or consists of (per) fluorinated hydrocarbons and/or (per) fluorinated silicones. In a particularly preferred embodiment said immiscible intermediate fluid comprises or consists of (per) fluorinated saturated hydrocarbons.

In a further preferred embodiment of the present invention said herein above mentioned immiscible intermediate fluid has a viscosity of less than about 0.1 Pa.s, a density of more than about 1100 kg/l, a boiling point higher than about 50°C and a solubility of water of less than about 1%.

In a further, particularly preferred embodiment of the present invention said herein above mentioned immiscible intermediate fluid is Fluorinert Electronic Liquid FC-40.

In yet another aspect the present invention relates to the use of a device as defined herein above for performing a sequencing reaction or for performing a nucleic acid synthesis reaction.

### BRIEF DESCRIPTION OF THE DRAWINGS

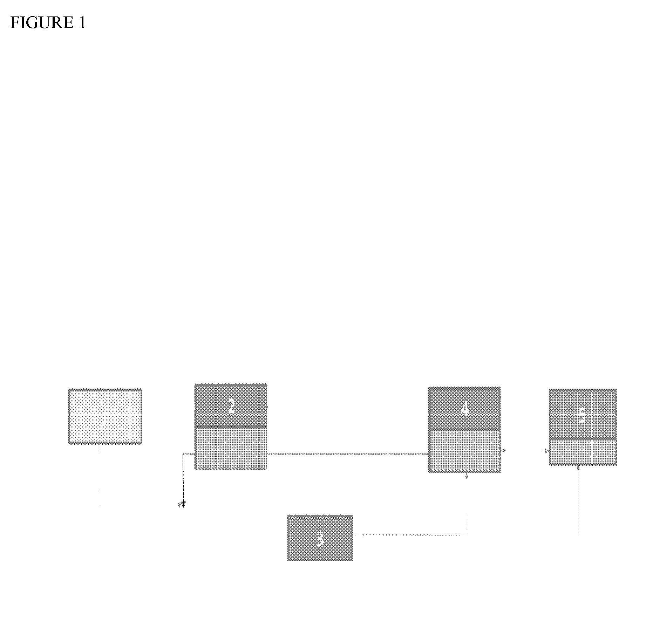

- Fig. 1: shows a fluidic scheme implementing the method of the invention in a side view with 1 = washing buffer (WB) reservoir, 2 = reagent solution (RS) and immiscible intermediate fluid (IF) reservoir, 3 = reaction chamber, 4 = reagent solution (RS) and immiscible intermediate fluid (IF) collection and regeneration chamber, 5 =waste chamber and X =valves.

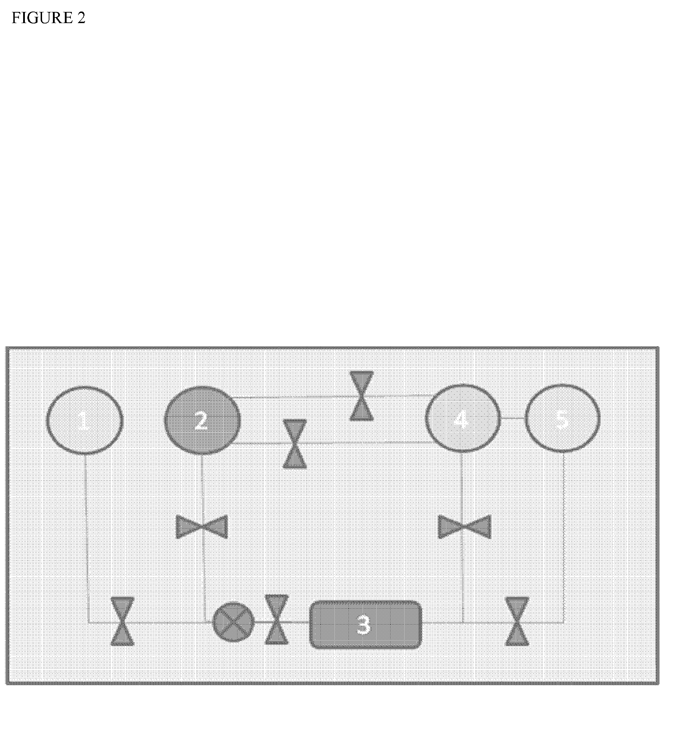

- Fig. 2: shows a fluidic scheme implementing the method of the invention in a top view with 1 = washing buffer (WB) reservoir, 2 = reagent solution (RS) and immiscible intermediate fluid (IF) reservoir, 3 = reaction chamber, 4 = reagent solution (RS) and immiscible intermediate fluid (IF) collection and regeneration chamber, 5 =waste chamber and X =valves.
- Fig. 3: shows the chemical structure of FC 40.
- Fig. 4: shows effects of FC-40 on hybridization signals.
- Fig. 5: shows effects of FC-40 on the end-repair PRC reaction.

### DETAILED DESCRIPTION OF EMBODIMENTS

The inventors have developed means and methods, which allow for performing molecular reactions with an increased speed and reduced overall costs based on a reuse of reagent solutions.

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" is considered to be a preferred embodiment of the term "comprising of'. If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" etc. relate to steps of a method or use there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

It is to be understood that this invention is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

As has been set out above, the present invention concerns in one aspect a method for performing molecular reactions in a device comprising the steps:
(a) introducing one or more reagent solution(s) and an immiscible intermediate fluid into the device, wherein the device comprises a substrate, on which chemically or biochemically recognizable entities are immobilized;
(b) performing molecular reactions between the immobilized chemically or biochemically recognizable entities and the reagent solution(s); or on the immobilized chemically or biochemically recognizable entities in the presence of the reagent solution(s);
(c) displacing the reagent solution(s) present on the substrate by the immiscible intermediate fluid;
(d) separating the immiscible intermediate fluid and the reagent solution(s), preferably in a collection and regeneration zone; and
(e) reusing the reagent solution(s) and/or immiscible intermediate fluid for one or more repetitions of steps (a) to (e).

The term "device" as used herein refers to a structure, e.g. a receptacle, chamber or container, or an instrument, which allows or is suitable for the performance of molecular reactions. The device may correspondingly be equipped with one or more inlet and/or outlet elements, it may comprise one or more surfaces, e.g. reactive surfaces or surfaces with specific functionality, it may comprise a reaction zone, a washing zone, a mixing zones, a waiting zone, a measurement zone, a waste zone, a reservoir zone, a recollection and regeneration zone or a recollection or regeneration zone etc. or any sub-portion or combination thereof. It may further comprise connections between these elements, e.g. tubes or joints; and/or it may comprise reservoirs and repositories for liquids, fluids, chemicals, ingredients, samples or any other entity to be used within the device.

Preferably, it may comprise a reaction zone. Such a "reaction zone" may be a closed entity comprising only inlet and/or outlet structures being of a minor size in comparison to the size of the zone and be understood as a "reaction chamber", or it may be an open structure being in free connection with further entities present in a device. The reaction zone may be suitable for allowing molecular reactions as mentioned, or may, in certain embodiment, be equipped with or comprise elements allowing a reaction to take place in said entity. To be suitable for allowing a reaction one or more parameters may be set or adjusted in a reaction zone. For example, the temperature in a reaction zone may be adjusted to a suitable value known to the person skilled in the art. The value may largely depend on the target molecule to be selected and the reaction or interaction type taking place and may differ in dependence of the reactant type, the reaction category, the envisaged speed, reaction end point considerations and further parameters known to the person skilled in the art.

Elements allowing a reaction to take place may be substrates, arrays of chemical, biochemical, biological or other entities, catalyst etc. Furthermore, a reaction zone may be composed of regions suited for measurement or movement activities, e.g. it may comprise a moveable surface allowing for a reduction of the enclosed space, and/or it may comprise electrically conductive zones or capacitor zones etc. and/or it may comprise one or more transparent surfaces allowing an optical detection. In certain embodiments the dimension and/or form of the reaction may be adapted to one of the above indicated functions. In a further embodiment the device may additionally or alternatively comprise one or more detection zones. This zone may be identical to the other zones, or may be separated from the other zones, e.g. the reaction zone or the mixing zone etc. A detection zone within the meaning o the present invention may comprise sensor or detector elements, e.g. for electrically, optically, olfactorially or audially detecting reaction products, reaction results or for checking whether reaction steps have been concluded or not. These zones may comprise, for example, electrically conductive zones or capacitor zones etc. or they may comprise one or more transparent surfaces allowing an optical detection, e.g. of reaction results such as, for example, the performance or intensities of labeling reaction etc.

In further embodiments of the invention the device may additionally or alternatively comprise heating modules or regulating units for controlling and/or regulating the temperature , e.g. a heating zone wherein the temperature may be kept constant at a desired value, or may be set to a desired value in dependence of a reaction type or reaction cycle etc. In further embodiments the device may additionally or alternatively comprise cooling modules, e.g. a cooling zone wherein the temperature may be kept constant at a desired value, or may be set to a desired value in dependence of a reaction type or reaction cycle etc. These zones may further also be equipped with suitable sensor elements allowing the measurement of temperature changes or temperature gradients.

Additionally or alternatively, the device may comprise units, elements or equipment allowing to change further parameters such as the presence of charged entities, the presence of ions, or may convey mechanical or shearing forces etc. For example, the element(s) may be suited to establish an electric or electrophoretic current, the element(s) may be suited to provide a specific pH or a specific presence of chemical or physical entities, e.g. the presence of certain acids, salts, solvents etc. and/or the element(s) may be suited to provide a strong medium movement. Any of the above mentioned additional facilities may be available in any of part of a device, e.g. in a reaction zone or reaction chamber.

In further specific embodiments the device may additionally or alternatively comprise modules allowing the detection of flow velocity, viscosity or density values, the transition of one state to another, the presence or absence of reagents, the presence or absence of intermediate immiscible fluids etc.

The term "molecular reaction" as used herein refers to a chemical, biochemical, biological or physical interaction, or any combination thereof, on a molecular basis or, in certain embodiments, on a macromolecular basis, preferably to a chemical or biochemical reaction. Also envisaged are in certain embodiments sub-molecular interaction, e.g. interactions involving parts or portions of the molecules, or single atoms or ions. The reaction may take place on the surface of the device, it may take place in entities within the device without having connection to its surface, or it may take place in both sectors of the device. For example, molecular reaction Preferably, the molecular reaction may take place in a reaction zone or a reaction chamber as mentioned herein above.

In step (a) one or more reagent solution(s) and an immiscible intermediate fluid are introduced into the device, wherein the device comprises a substrate, on which chemically or biochemically recognizable entities are immobilized. The term "reagent solution" as used herein refers to a liquid or fluid comprising one or more reactants necessary for or allowing an interaction between the components of the solution themselves, or between the components of the solution and structures, elements or forms of the device, e.g. structures, elements or forms of a reaction zone or reaction chamber. The reagent solution may, for example, be an aqueous solution comprising chemical, biochemical or biological entities, such as nucleic acids or fragments thereof, peptides, polypeptides, e.g. antibodies, enzymes etc., nucleotides, chemical labels, salts or ion of specific concentrations, one or more buffers or buffer components etc., or macromolecular structural elements such as moveable beads, polymeric structures etc. Furthermore, the reagent solution may comprise optically detectable dyes, which may for example be used for a detection of a transition between reagent solution and an immiscible intermediate fluid, or between two or more types of reagent solution. According to the present invention, a single reagent solution may be employed. Alternatively, 2, 3, 4, 5, 6, 7, 8, 9, 10 or more different reagent solution may be employed. The term "different reagent solution" as used herein refers to any difference in the above indicated ingredients, e.g. the presence or absence, or differences in the concentration of nucleic acids, peptides, polypeptides, ions, salts, buffer components, dyes etc. A difference in concentration may, for example, be an increase or decrease of about 5%, 10%, 20%, 50%, 100%, 500%, 1000% or more or any value in between. Furthermore, a difference may also be a difference in temperature of the reagent solution, in its viscosity, density, electrical conductance or in flow parameters which would be known to the person skilled in the art. Different reagent solutions may, for example, comprise an annealing solution, a binding solution, a de-naturation solution, a blocking solution, a de-blocking solution etc. or any combination thereof.

The term "immiscible intermediate fluid" as used herein refers to a fluid which is substantially not miscible or entirely immiscible with a reagent solution as defined herein above. A "substantially not miscible" intermediate fluid may comprise up to about 0.01, 0.02, 0.05, 0.07, 0.08, 0.09, 0.1, 0.2, 0.3, 0.4, 0.5, 0.6, 0.7, 0.8 or up to 1% of a different fluid, in particular of a reagent solution, e.g. of a aqueous reagent solution as mentioned herein. Preferably, an immiscible intermediate fluid comprises a proportion of less than 1%, more preferably less than 0.5%, even more preferably less than 0.1% of water or of a aqueous solution. Typically, the immiscible intermediate fluid forms a separate phase when being in contact with a reagent solution, e.g. an aqueous solution as defined herein. The separation between the immiscible intermediate fluid and the reagent solution may preferably be substantially complete or complete, e.g. leading to a separation of the immiscible intermediate fluid and the reagent solution of at least 99%, 99.5%, 99.9%, 99.99%, 99.999%, 99.9999% or 100%. In specific, preferred embodiments of the present invention said immiscible intermediate fluid shows an insolubility of components of the reagent solution in the intermediate fluid, or a substantial insolubility of components of the reagent solution in the intermediate fluid. The term "insolubility of components of the reagent solution" as used herein means that the composition of the reagent solution does not change upon contact with the intermediate fluid. The term "substantial insolubility of components of the reagent solution in the intermediate fluid" as used herein means that the intermediate fluid may comprise up to about 0.01, 0.02, 0.05, 0.07, 0.08, 0.09, 0.1, 0.2, 0.3, 0.4, 0.5, 0.6, 0.7, 0.8 % or up to 1% of one or more components of the reagent solution. In further embodiments said insolubility in particular refers to surface areas, where the composition of reagent solution may be substantially unchanged, thus allowing a reuse. In a particularly preferred embodiments of the invention, the ratio of reactive sites on a substrate, e.g. of chemically and biochemically recognizable entities, may be such that there is a large excess in solution.

An immiscible intermediate fluid may comprise molecules of a single chemical identity, e.g. a single structure or conformation, and/or of a single physical property, e.g. single boiling point, a single viscosity, a single density, a single electrical conductivity, or it may comprise two or more molecule types or categories, e.g. molecule types of different chemical identity. Preferably, such different molecule types have similar or comparable physical properties, e.g. similar boiling points, or similar viscosities, similar densities, or electrical conductivities. Further preferred are molecule types whose chemical and/or physical properties are similar in way allowing a combination of both molecules to provide a combined immiscible intermediate fluid, having an averaged boiling point, viscosity or density.

The term "intermediate" as used herein in the context of "immiscible intermediate fluid" refers to the fluid's function as detaching or separating the one or more reagent solution(s) or any other fluid entity from one another. For example, if two different reagent solutions or fluid entities are provided, e.g. in the form of droplets, fluid units or plugs, said intermediate immiscible fluid according to the present invention may be in between these reagent solution units or fluid units, e.g. filling in the space between two droplets or plugs. Thus, the one or more reagent solution(s) according to the present invention may be detached by the immiscible fluid, which hence constitutes an intermediate fluid between said reagent solution(s) or between any other type of fluid ingredient in the device which is not miscible with said immiscible intermediate fluid. By performing this activity, immiscible intermediate fluids become separated in the form of fluid units, droplets or plugs themselves. In a specific embodiment of the present invention units of identical reagent solutions may be passed through or over a reaction zone, which are detached or separated by immiscible fluids. Preferably, the detachment leads to a complete immiscibility or complete non-mixture of the first reagent solution with the immiscible fluid and/or of the first reagent solution with the second or subsequent reagent solution. In further specific embodiments such reagent solution units may comprise different ingredients, different concentrations of ingredients, may have different temperatures, different viscosity, different electrical conductivity, be differently colored, have a different pH, different enzyme concentration, show the presence or absence of blocking materials, detergents, ions, salts or have any other suitable parameter difference. Such further parameters would be known to the person skilled in the art or can be derived from suitable textbooks.

In further specific embodiments of the invention, a first reagent solution introduced into the device may cause a molecular modification of device portions, e.g. of substrate regions, in particular of regions of reaction zones or reaction chambers as mentioned herein. Alternatively, such a modification may also be caused by subsequently introduced reagent solutions. A corresponding molecular modification may, for example, be the occupation of binding sites, interaction domains, active sites or domains, or the wetting of surface regions or substrates, preferably with a thin film of liquid, more preferably with a thin water film. Being modified in a manner as depicted above, the presence of said immiscible intermediate fluid may not entirely provide a non-miscibility of portions of a first and a second unit of reagent solution, e.g. since the factors or entities bound to binding sites, or the thin liquid film may be kept in situ and hence be mixed with a second or subsequent reagent solution.

In further specific embodiments, an immiscible intermediate fluid may comprise additional ingredients, i.e. be comprised of more than one molecule type or category, but comprise compounds such as a surfactant, a detergent, a dye or a suitable stabilizer.

The term "introducing" as used herein refers to the provision of a reagent solution(s) and/or immiscible intermediate fluid in the above mentioned device. This provision may be carried out by injecting said reagent solution(s) and/or immiscible intermediate fluid into the device. For example, in a mixing zone or a T-shaped zone of a device a reagent solution may be injected from an outside or inside reservoir, or from a syringe or any other suitable receptacle. Similarly, in such a mixing zone or T-shaped zone of a device an immiscible fluid may be injected from an outside or inside reservoir, or form a syringe or any other suitable receptacle. Preferably, the introduction of these entities takes place rotationally, or alternating.

The term "substrate" as used herein refers to any suitable substrate known to the person skilled in the art. The substrate may have any suitable form or format, e.g. it may be flat, curved, e.g. convexly or concavely curved towards to zone where an interaction takes place, it may be curled or comprise a wavelike format. It may also be organized in round shape structures, or it may be organized in the form of bead-like elements or beads, which may, for example, be arranged in an array. The beads may overly a substrate ground or be fixed in the reaction area by connector elements like rods, linkers etc., or may comprise magnetic particles comprising capture molecules, be covered by specific blocking reagents etc.

In further, specific embodiments, coated beads as known to the person skilled in the art may be used. In additional embodiments, the substrate may comprise or consist of micro-structured surfaces, coatings, or mobile carriers which may preferably be kept in the reaction zone or reaction chamber, or bead-like elements, however of different shape.

Typically, the substrate is a solid support, i.e. comprising support material which is mainly of non-liquid consistence and thereby allows for an accurate and traceable positioning of the entities on the support material. An example of a substrate is a solid material or a substrate comprising functional chemical groups, e.g. amine groups or amine-functionalized groups. Further examples of a substrate envisaged by the present invention is a porous support material or porous substrate such as nylon, e.g. Nytran N^{®} or Nytran SPC^{®} or Biodyne C^{®}. A further typical support material or substrate type is a non-porous substrate. Preferred among non-porous substrates are glass, silicone, fused silica, coated with a silane layer with functionalized groups, like, polyethyleneglycol, amine, epoxide, isothiocyanate, etc., poly-L-lysine coated material, nitrocellulose, polystyrene, cyclic olefin copolymers (COCs), cyclic olefin polymers (COPs), polypropylene, polyethylene and polycarbonate. Further examples of substrates, which are in particular useful for the immobilization of peptide/polypeptide entities, include calcium alginate or modified versions thereof. Typically, nucleic acids may be coupled to a polymeric or inorganic surface substrate, e.g. as mentioned herein above, with covalent coupling, whereas peptide, polypeptide or protein entities may be coupled to a polymeric or inorganic surface substrate, e.g. as mentioned herein above, with or without covalent coupling.

The term "recognizable entity" as used herein refers to an entity, in particular a molecule or macromolecule, which is able to interact with other molecules, buffers, ions, salts or other chemicals and thereby results in a distinction from the starting situation or from a previous state, e.g. by the building of covalent bonds, the occupation of active centers, the binding to suitable regions or domains, the change of conformations or superficial structures, the incorporation of chemical entities, e.g. nucleotides, dyes, or any other distinction known to the skilled person.

A "chemically recognizable entity" as used herein refers to a chemical group, e.g. a reactive group of an organic molecule, or an inorganic element or molecules, or a biochemical or biological molecule, which is suitable for chemical interactions, reactions, binding interactions or any other type of interaction which provides a situation which is chemically distinguished form the starting situation, or from a previous state. For example, such entities may be reactive for specific chemical reactions, or they may be able to allow chemical binding interactions, catalyses, transformations, or the production of chemical products. The term "biochemically recognizable entity" as used herein refers to substantially macromolecular interactions, e.g. binding interactions between ligands and corresponding receptors, or the interaction between an enzyme and its substrate, or a blocking reagent etc. These interactions largely involve biochemically or biologically relevant entities, such as nucleic acids, peptides, polypeptides, proteins, antibodies, enzymes and their substrates, cofactors, binding factors etc. In certain embodiments chemically and biochemically recognizable entities may be similar or essentially identical, or a biochemical interaction may be based on chemically recognizable entities or vice versa.

The term "immobilized" as used herein refers to the association of the chemically or biochemically recognizable entities to a supportive substrate via molecular interactions which position said entities at a specific area of the substrate and concomitantly impede a detaching of the entities, e.g. during washing, rinsing displacing or interaction steps etc. Typically, such molecular interactions are based on covalent chemical bonds between structural elements or functional groups of the support material and the chemically or biochemically recognizable entities to be immobilized, e.g. corresponding functional groups of nucleic acids, peptides/polypeptides, carbohydrates, or modified versions thereof, or combinations thereof. Such functional groups and the employment of suitable chemistry for their use in immobilization processes would be known to the person skilled in the art.

The immobilization may, in one specific embodiment, be carried out by crosslinking chemically or biochemically recognizable entities by heat or light, i.e. by forming molecular interactions or bonds that link both structural elements together under the influence or driven by the energy provided by an energy source like heat or light, or via a chemical immobilization. Typically, an immobilization via crosslinking by heat is carried out via drying and subsequent baking of chemically or biochemically recognizable entities on a substrate at certain temperatures. This approach is preferably used for the immobilization of nucleic acids. Drying and baking are believed to result in molecules becoming attached to the substrate by hydrophobic interaction. This procedure can be classified as a sub-form of physical adsorption. The term "physical adsorption" relates to a process involving initial separation and attraction steps, whereby the chemically or biochemically recognizable entities comes into proximity with the reactive groups, which are based on physical adsorptive processes. The adsorption of a biomolecule, e.g. a nucleic acid, onto a solid support may take place with practically any support material, since it has been observed that any such support material will interact with almost any surface.

Typically, the level of interaction between support material and chemically or biochemically recognizable entities to be immobilized varies depending on the nature and form of the support material and the size and chemical/physical/biological properties of the chemically or biochemically recognizable entities. The interaction is typically a five-stage procedure, comprising the steps of (i) transport of the chemically or biochemically recognizable entities to the surface of the substrate, (ii) adsorption to said surface, (iii) rearrangement of the adsorbed chemically or biochemically recognizable entities, (iv) potential desorption of the adsorbed chemically or biochemically recognizable entities and (v) transport of the desorbed chemically or biochemically recognizable entities away from the surface. Although the procedure implies, to a certain extent, that the potential for desorption is inherent, the binding is typically irreversible, depending on the size of the chemically or biochemically recognizable entities. The term "size of the chemically or biochemically recognizable entities" within the context of adsorption interactions relates to the number of binding sites that are present. Although any one binding site may, in principle, dissociate from the surface of the substrate at any time, the effect of a large number of binding sites is that the molecule comprising chemically or biochemically recognizable entities as a whole will remain bound. By applying energy in the form of heat, e.g. at a temperature of about 40 to 150°C, preferably 50 to 120°C, more preferably 60 to 110°C, even more preferably 70 to 100°C and most preferably 80 to 90°C, the physical adsorption of the chemically or biochemically recognizable entities to the support material may be enhanced and the time necessary for an efficient immobilization may be shortened. The crosslinking by heat may be carried out for any suitable period of time known to the person skilled in the art, e.g. 2 min to 12 hours, preferably 1 hour to 3. The crosslinking by heat or baking may be carried out by any suitable means known to the person skilled in the art, for example a drying chamber or an oven. In addition to the temperature, also other parameters like humidity, aeration or ventilation may be adjusted to suitable values known to the person skilled in the art. The crosslinking by heat or baking may also be combined with other forms of immobilization like crosslinking by light or chemical immobilization.

Crosslinking by light is typically performed by applying light of a typical wavelength, e.g. in a range of 150 to 550 nm, preferably in a range of 200 to 500 nm to chemically or biochemically recognizable entities in order to induce an interaction between the chemically or biochemically recognizable entities and support material. Typically, the induced interaction between the chemically or biochemically recognizable entities and the support material is a covalent binding of the chemically or biochemically recognizable entities, e.g. a nucleic acid, to the material. Crosslinking by light may, for example, be carried out by using UV light. In the case of nucleic acids, the linkage proceeds through the bases of a nucleic acid molecule, e.g. thymine, guanine, adenine, cytosine or uracil residues, which react with corresponding and suitable functional chemical groups on the support material, as known to the person skilled in the art. The presence and number of functional chemical groups on or inside the support material may be controlled and adjusted via suitable chemical activation processes. Such activation processes may, for instance, provide specifically localized functional groups on or within a support material and facilitate a specific interaction between the chemically or biochemically recognizable entities and the material within the context of these localized functional groups. The presence and number of functional group on or inside the support material may also have an influence on the orientation and freedom of the immobilized chemically or biochemically recognizable entities.

A "chemical immobilization" as mentioned herein may be an interaction between the support material and the chemically or biochemically recognizable entities based on chemical reactions. Such a chemical reaction does typically not rely on the input of energy via heat or light, but can be enhanced by either applying heat, e.g. a certain optimal temperature for a chemical reaction, or light of certain wavelength. For example, a chemical immobilization may take place between functional groups on a support material and corresponding functional elements of the chemically or biochemically recognizable entities. Such corresponding functional elements in the chemically or biochemically recognizable entities may either be as part of the chemical inventory of said molecules, or be additionally introduced. An example of such a functional group is an amine group. Typically, the chemically or biochemically recognizable entities to be immobilized, e.g. a nucleic acid or a peptide/polypeptide, comprises a functional amine group or is chemically modified in order to comprise a functional amine group. Means and methods for such a chemical modification would be known to the person skilled in the art.

Alternatively, the chemically or biochemically recognizable entities may be synthesized directly on the substrate. Suitable methods for such an approach are known to the person skilled in the art. Examples in the ambit of nucleic acids are manufacture techniques developed by Agilent Inc., Affymetrix Inc., Nimblegen Inc. or Flexgen BV, wherein Affymetrix Inc. for example, uses illumination through a reticle, while Nimblegen Inc. uses pixilated mirrors and Agilent Inc. offers a technique of printing the 4 different nucleotides in the appropriate position. Flexgen BV uses a scanning laser beam. Typically, lasers and a set of mirrors that specifically activate the spots where nucleotide additions are to take place are used. Such an approach may provide, for example, spot sizes of between 5 and 100 µm, e.g. around 30 µm or larger. Chemically or biochemically recognizable entities, for instance nucleic acids, may accordingly have a length of up to about 80 nucleotides. In a different, also envisaged technique the chemically or biochemically recognizable entities may be deposited by using a non-contact inkjet printing process. In specific embodiments of the present invention nucleic acids, preferably oligomers, may be deposited uniformly onto specially-prepared glass slides. This in situ synthesis process may typically produce 60-mer length oligonucleotide probes, either base by base in a synthetic approach or by printing presynthesized oligomers and coupling them to the surface covalently.

For the immobilization of a peptide, polypeptide or protein, for example the immobilization of an enzyme, a covalent bonding to a substrate, e.g. in the form of a matrix through a chemical reaction is preferably envisaged. This technique may be complemented or supported by the employment of suitable spacer molecules. An example of such a spacer molecule is polyethylene glycol, which typically reduces the steric hindrance by substrate elements.

In step (b) molecular reactions between the immobilized chemically or biochemically recognizable entities and the reagent solution(s) are performed; or molecular reactions are performed on the immobilized chemically or biochemically recognizable entities in the presence of the reagent solution(s). The term "performing" as used herein refers to a contacting of one or more reagent solutions with an immobilized chemically or biochemically recognizable entity as defined herein above in a zone or region of the device where said immobilized chemically or biochemically recognizable entity are located, preferably in a reaction zone or reaction chamber. The contacting may be carried out for any suitable period of time, e.g. 1, 5, 10, 30 sec, 1 min, 2, min, 5 min, 30 min 1 h, or several hours, or even days, or any time in between these values. The period of time may depend on the type of molecular reaction to be carried out, the amount and/or concentration of ingredients in the reagent solution(s), e.g. the concentration of enzymatic substrates, nucleotides, labels, dyes etc., the temperature used, the absence or presence of blocking reagents, the kinetics of the reaction and any further parameter known to the person skilled in the art. The contacting may further be carried out under further suitable conditions, e.g. at a specifically set pH or a specific range of pH, at a specific temperature or a specific temperature range, at certain ingredient concentrations, e.g. at specific ionic concentrations, specific concentrations of polypeptides/peptides in the reaction solution, preferably at specific enzyme concentrations etc. Such conditions would be known to the person skilled in the art and may be adapted to the reaction type and/or envisaged reaction outcome. In specific embodiments of the present invention, the concentration of enzymes, more preferably the concentration of DNA polymerase, may be set to a value substantially above the currently used amount of DNA polymerase in sequencing reactions, e.g. 1.5 times, 2 times, 5 times, 10 times, 20 times or 50 times as high as currently used. Similarly increased concentrations of other ingredients, e.g. nucleotides, substrates etc. may in specific embodiments also be used.

The performance of the molecular reaction may, in certain embodiments of the present invention, be controlled by external activities. For example, the temperature may be set, increased or decreased by using suitable heating or cooling modules as mentioned herein above. Furthermore, the pH may be measured, and/or changed by suitable pH equipment present in the device, preferably in the reaction zone or reaction chamber.

The performance of a molecular reaction, preferably of a complete molecular reaction, may depend on the presence of one reagent solution or may be based on the employment of two or more reagent solutions. For example, a first reagent solution may provide an ingredient necessary for a first reaction step, or a preparation step, e.g. a deblocking step, or a blocking step etc. A second or subsequent reagent solution may provide an ingredient necessary for a second or subsequent reaction step, e.g. an ingredient for an interaction such as a specific enzyme, or a substrate for an enzyme, or specific label or dye etc.

The performance of said molecular reaction may be carried out between the immobilized chemically or biochemically recognizable entities, e.g. nucleic acids, peptides/polypeptides, carbohydrates and the reagent solution(s), which may typically lead to a chemical or biochemical modification of the immobilized entities, e.g. an addition of chemical groups to said immobilized entities and/or a chemical or biochemical modification of the reagent solution(s). Alternatively, the performance of said molecular reaction may be carried out on the immobilized chemically or biochemically recognizable entities in the presence of the reagent solution. For example, said reagent solution(s) may provide catalytic ingredients, e.g. enzymatic activities being necessary for the performance of a reaction on the immobilized chemically or biochemically recognizable entities, however without modifying or without substantially modifying the reagent solution. The molecular reaction may preferably be initiated and/or terminated by movement activities leading to a contacting of said chemically or biochemically recognizable entities and said reagent solution(s) and/or the termination of said contacting of said chemically or biochemically recognizable entities and said reagent solution(s). Such an initiation or termination may be based on a movement of the reagent solution(s), in particular of reagent solution units or plugs as described herein above, through a reaction zone or reaction chamber. Preferably the movement is carried out by using an immiscible intermediate fluid according to the present invention. Thus, by controlling the movement, e.g. the flow velocity, or by starting, speeding up, slowing down, pausing or stopping the movement at specific zones or regions of the device a molecular reaction may be controlled, its velocity may be modified, the amount of reaction products may be changed etc. In specific embodiments of the present invention the initiation and in particular the termination of said molecular reactions may be made dependent on the outcome of measurements of reaction products. For example, such a measurement-dependent termination process may be implemented by a microelectronic circuit, e.g. a computerized module, coupling the presence or absence of suitable parameters to a reaction state, a reaction stage or the reaction progress. In step (c) the reagent solution(s) present on the substrate are displaced by the immiscible intermediate fluid of the present invention. The term "displacing" as used herein refers to an expulsion of a reagent solution unit or plug, e.g. introduced into the device as described herein above, from specific zone or region of the device. Preferably, the displacement may lead to an expulsion of the reagent solution(s) form the substrate of the reaction zone of the device. In addition or alternatively, a displacement of a reaction solution, or of any other solution unit non-identical and non-miscible with the immiscible intermediate fluid may take place not only at the reaction zone/in the reaction chamber, but at any other site or region of the device, e.g. in a tubing, in a connection, in waiting or repository zones etc.

In specific embodiments of the present invention said displacing may be a complete, or substantially complete displacing, e.g. leading to a 99%, 99.9%, 99.99% or 100% removal of the reagent solution or of any other solution from the substrate, e.g. the substrate of the reaction zone, or a complete, or substantially complete displacing from any other site or region of the device. In further, alternative embodiments the displacing may not be complete or substantially complete. Instead by carrying out a displacing activity, a certain amount of previous present solution, in particular of previously present reagent solution may be kept on the substrate. In preferred embodiments a film of liquid, e.g. of reagent solution, or an aqueous film, may be kept on the substrate and not be displaced by said immiscible intermediate fluid. Concomitantly, further ingredients of the reagent solution(s) or of any other solution or fluid may be kept on the substrate during said displacement activity.

In further preferred embodiments of the present invention, immiscible intermediate fluid residuals or films may not be kept at substrate surfaces or at any other region or site of the device. A displacement of immiscible intermediate fluid residuals may accordingly be achieved by following units of reagent solution passing through a certain zone or region of the device.

A displacing step according to the presently described method may lead to a movement of the reagent solution(s) and also of the displacing immiscible intermediate fluid to a region or portion of a device remote from the region or portion of the device where it was previously located. Such a movement, which may be carried out by any suitable means known to the person skilled in the art, may transport a reagent solution unit or plug, as well as the immiscible intermediate fluid towards different zones of a device, e.g. a waiting zone, a measurement zone, a waste zone, a reservoir zone, a recollection and regeneration zone or a recollection or regeneration chamber etc. The movement may be controlled or influenced by suitable means, e.g. valves, gates, locks, junctions, confluences etc. A control may be carried out, for example, mechanically, or electronically or by any other suitable means.

In step (d) the immiscible intermediate fluid and the reagent solution(s) are separated. The term "separation" or "separating" as used herein refers to an activity to be performed on a mixture of immiscible intermediate fluid units and reagent solution units. The separation may, in further embodiments, also be carried out between any other liquid or fluid units present in the device. Accordingly, the separation may constitute a disintegration of a sequence or order of liquid or fluid units present in a device, wherein a reagent solution or other solution is preceded and/or followed by the immiscible intermediate fluid according to the present invention. This separation preferably leads to a collection of identical or similar liquid or fluid units, e.g. a collection of immiscible intermediate fluid, a collection of a reagent solution, a collection of a specific type of reagent solution, or a collection of any other type of fluid unit present in the device.

In a preferred embodiment of the present invention said separation may be performed in a collection and regeneration zone. The term "collection and regeneration zone" as used herein refers to a portion of the device, or an element being located outside of the device, where reagent solution(s) and immiscible intermediate fluid(s) are caught and collected. Typically, such a collection and regeneration zone may have the form of a collection and regeneration chamber or of a collection and regeneration container or receptacle. Subsequent to the collection of said reagent solution(s) and immiscible intermediate fluid(s) a separation may be carried out, e.g. by any suitable means such as the employment of gravitational forces, the employment of centrifugation, electrical separation approaches, electrophoretic separation techniques, density gradient separation etc.

Being separated, said reagent solution(s) and immiscible intermediate fluid may be transported back to zones of the device where said above mentioned molecular reaction(s) take place, e.g. by a rechanneling from suitably positioned outlets in a collection and regeneration zone to the reaction zone, or by any other suitable recycling technique.

A separation as described herein may allow a separation between two or more than two fluids or liquids, e.g. between two or more different immiscible intermediate fluids and a reagent solution, or between two or more reagent solution(s) or other solutions and an immiscible intermediate fluids. A prerequisite for a separation of more than two of these fluids is the generation of different phases after any of the above mentioned separation approaches.

In a preferred embodiment, said separation is a complete or substantially complete separation. For example, the separation between two or more fluids, in particular between a reaction solution and an immiscible intermediate fluids according to the present invention may be 90%, 95%, 99%, 99.9%, 99.99%, 99.999% or 100%. In case a separation procedure as mentioned above is carried out and a non-separable zone remains between two or more liquid phases, this non-separable zone may be wasted, or it may be subjected to a different separation procedure, e.g. an enhanced separation by centrifugation after a gravitational procedure being performed in the first place etc.

In a further specific embodiment of the present invention upon separation of said reagent solution(s) and said immiscible intermediate, the reagent solution(s) and/or the immiscible intermediate fluid may be regenerated, e.g. by an adjustment of pH, an adjustment of viscosity, an adjustment of the concentration of ingredients etc. before they are transported back to zones of the device where said above mentioned molecular reaction(s) take place, e.g. by a rechanneling from suitably positioned outlets in a collection and regeneration zone to the reaction zone, or by any other suitable recycling technique.

In specific embodiments of the present invention said separation may skip a collection or recollection step and lead to a direct spatial separation of said immiscible intermediate fluid, in particular of said immiscible intermediate fluid units or plugs, from a said reagent solution(s), in particular said reagent solution units or plugs, or any other solution or fluid plugs, allowing their direct reuse by suitable means such as rechanneling activities etc. For example, a redirection, branching off or deflection of immiscible intermediate fluid units or plugs, and/or of reagent solution units or plugs, into separated channels, tubes or zones may be implemented in a device according to the present invention.

In step (e) reagent solution(s) and/or the immiscible intermediate fluid are reused. Thus, in one embodiment, following disintegration of a sequence or order of immiscible intermediate fluid and reagent solution(s) units or plugs in a device, e.g. in a channel section of the device, according to step (d), as explained herein above, said separated fluid units may be employed for a second or subsequent use in the presently claimed method. Said fluid units may be either rejoined before reusing them, or they may be used as units in their present form. Due to the non-miscibility of the immiscible intermediate fluid and the reagent solution(s) said reagent solution(s) or reagent solution units will according to the present invention neither be diluted nor polluted or contaminated with any other fluid or liquid present in the device, e.g. with different reagent solutions, or with washing or rinsing buffers etc. Typically, a reagent solution comprising a certain amount of ingredients may be reused for several times, e.g. for 1, 2, 3, 4, 5, 6, 7, 8, 9, 10, 15, 20, 30, 40, 50 or more times, e.g. by rechanneling it to a zone of the device where a molecular reaction takes place, e.g. a reaction zone or reaction chamber as defined herein above. In certain embodiments, the reuse of a reagent solution may be made dependent on the concentration of one or more ingredients, which may, for example, be determined by suitable means in specific portions of the device. Alternatively, a reuse may be made dependent on the quality and/or velocity of a molecular reaction according to the present invention, e.g. determined according to a measurement of the reaction products etc. as described herein above. In further embodiments, a reaction solution may be provided with missing ingredients, e.g. the concentration of ions, proteins, e.g. enzymes, nucleotides, dyes or any other compounds may be raised after a certain amount of time or a certain number of reuses, or after a measurement of the concentration of said ingredients or of the reaction outcome as described above. Upon a decrease of suitability, i.e. a decrease concentration of ingredients, a decrease of quality and/or velocity of a molecular reaction, the reagent solution(s), e.g. certain reagent solution units, may be wasted. Such wasted units may preferably be replaced by a reintroduction of reagent solution, e.g. reagent solution units.

Said above mentioned immiscible intermediate fluid may be reused for several times, e.g. for 5, 6, 7, 8, 9, 10, 15, 20, 30, 40, 50, 100, 150, 200 or more times, e.g. by rechanneling it to a zone of the device where a molecular reaction takes place, e.g. a reaction zone or reaction chamber as defined herein above, or to a zone of the device where to introduction of said fluid takes place, as defined herein above. A reuse of the immiscible intermediate fluid may be made dependent on the non-miscibility quality of the fluid, its viscosity, its density and further suitable parameters known to the person skilled in the art. These parameters may be measured in the reaction zone or in any other suitable measurement zone of the device. Upon a decrease of suitability, i. e. a decrease of the non-miscibility quality, a change of viscosity or density, the immiscible intermediate fluid, e.g. certain non-working or qualitatively decreased immiscible intermediate fluid units, may be wasted. Such wasted units may preferably be replaced by a reintroduction of immiscible intermediate fluid.

In a specific embodiment of the present invention the above mentioned step (d) may be skipped and a sequence of units of regent solution(s) preceded and/or followed by units of immiscible intermediate fluid may, after having passed a reaction zone or reaction chamber, be directly channeled back to said reaction zone or reaction chamber in the original sequence, or in a modified sequence, e.g. by the introduction of further units of a solution and/or of an immiscible intermediate fluid. Reagent solution(s) and/or immiscible intermediate fluids may preferably be reused by repetition of method steps (a) to (d) as described herein above. In alternative embodiments of the present invention, a reuse may be based on a repetition of only a sub-set of these steps, e.g. a separation step may be skipped and the sequence of fluid units may be directly rechanneled to a reaction zone, or to any other zone of the device.

A repetition of the steps as described above may be carried out for any suitable number of times. This may depend on the type and number of reactions to be performed, the quality and concentration of ingredients in a reaction solution, the velocity of the reaction steps, flow parameters in the device or any other suitable parameter known to the person skilled in the art. For example, the method steps may be repeated 1, 2, 3, 4, 5, 6, 7, 8, 9, 10, 15, 20, 30, 40,41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74,75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 115, 120, 125, 130, 135, 140, 145, 150, 155, 160, 170, 180, 190, 200 or more times.

In further embodiments, reagent solution(s) and/or immiscible intermediate fluids may be collected or stored in the device, in device zones or chamber or external receptacles, while a substrate on which chemically or biochemically entities are immobilized is replaced or modified. Thus, the present invention also envisages a reuse of said reagent solution and immiscible intermediate fluid for the performance of reactions on more than one substrate or substrate type.

In another preferred embodiment of the present invention the herein above described method may additionally comprise the step of washing said substrate with a washing buffer. The washing of said substrate may be carried out with a suitable washing buffer, e.g. an aqueous liquid comprising one more detergents, and/or a buffering component, and/or a salt, and/or a stabilizer. For example, a washing buffer may comprise TRIS-Base, Tween, formamide, NaCl and a stabilizer, having a pH of about 7.0. Further examples of suitable washing buffer comprise 10X PBS (0.1M PBS, pH 7.4), 20X PBS, (0.2M PBS, pH 7.4), 10X PBS-Tween 20 (0.1M PBS, 0.5% Tween 20, pH 7.4) or 20X PBS-Tween 20 (0.2M PBS, 1% Tween 20, pH 7.4). Further washing buffers and their use in specific reactions or situations would be known to the person skilled in the art, or can be derived from suitable textbooks.

Said washing buffer is preferably detached from the above mentioned reagent solution(s) by the immiscible intermediate fluid as mentioned herein above. Thus, after having displaced a reagent solution unit or two or more reagent solution units, e.g. of different ingredients or function as described above, by an immiscible intermediate fluid a washing buffer may be introduced into a device and transported through or over the regions of said device which comprise the above mentioned substrate. The washing buffer unit may in a particularly preferred embodiment of the present invention be followed by an immiscible intermediate fluid, e.g. a further immiscible intermediate fluid unit. This following immiscible intermediate fluid unit may subsequently displace said washing buffer from said substrate, e.g. form a substrate located in a reaction zone or reaction chamber. The displacement may preferably be complete or substantially complete.

In further preferred embodiments of the present invention said washing buffer may be reused, by a separation procedure as described herein above. Alternatively, said washing buffer may be wasted, e.g. by removing it after a separation process or a redirection process as defined herein above.

In further specific embodiments of the present invention, a washing buffer activity may be enhanced by reverting the transport direction, i.e. the movement direct of the fluid units, such a reversal of the flow direction within the reaction zone may improve a washing activity. In a further specific embodiment of the present invention the flow rate of the washing buffer may be adjusted, e.g. in order to achieve a suitable stringenc, preferably in combination with the setting of certain temperature.

In another preferred embodiment of the present invention the herein above described method may additionally comprise the step of measuring the state or outcome of the described molecular reaction. The term "measuring the state or outcome of the molecular reaction" as used herein refers to a determination of end products and/or intermediate products derived from a molecular reaction as described herein. For example, the presence of labeled or non-labeled molecules, the presence or absence of colors or dyes in the solution, the change of pH, the concentration of nucleic acids, peptides, polypeptides etc., electrical conductance or any other suitable parameter may can be measured. This measurement may be carried out in a reaction zone or chamber as described herein above, and/or in a specific measurement or detection zone of a device according to the present invention. The site of measurement may, for example, depend on the reaction type, e.g. if the reaction takes place in or on immobilized entities on the substrate leading to their detectable modification, or if it takes place in the reagent solution, leading to its detectable modification. If the immobilized entities are modified, a measurement may be carried out in situ, whereas a measurement of the stat or outcome of a molecular reaction in a reagent solution may either take place in a reaction zone or reaction chamber, or at any other site of the device, e.g. in a specific measurement or detection zone separated from the reaction zone.

A corresponding measurement may be carried out by optical means. For example, a CCD camera or other suitable optical equipment may be used in order to detect optical changes in a solution or at a substrate, and/or in order to determine the quality of such an optical change. Alternatively or additionally a corresponding measurement may be carried out by electrical means, e.g. based on the determination of conductivity, capacity or ionic strength, pH, and/or electrophoretic parameters at a site or in zone of the device according to the present invention. Corresponding measurement modules may be connected to an integration station, e.g. a computer, a computerized module or a microelectronic device allowing an assessment of the stage, velocity, or performance of a molecular reaction. Such an integration station may further be equipped with suitable software or programs allowing such a control activity and assessment. Such an assessment may further be used for applying corrective steps, e.g. the increase or decrease of velocity or flow parameter, the introduction of further reagent solution ingredients, the increase or decrease of the reaction temperature, the increase or decrease of time period allocated for reaction steps etc.

In a particularly preferred embodiment of the present invention corresponding optical measurements may be used for detecting a sequence in the case of a sequencing reaction, e.g. via the color of the luminescence or the presence or absence of a response at a certain spot.

In yet another preferred embodiment of the present invention said displacing step (c) of the above described method is to be performed by pneumatic fluid driving, peristaltic actuation, piston and actuated membrane driving or the application of vectored vacuum.

A pneumatic fluid driving may be implemented, for example, by using airflow or gasflow modules at different positions of the device. The modules may pump air or gas into the device. Corresponding modules and device sections may be provided in tubules or connective portions of the device. It is preferred to use a gaseous compound which is substantially immiscible with the reagent solution and/or the immiscible intermediate fluid, and/or which can be removed therefrom easily. Further details and variations of this technique would be known to the person skilled in the art or could be derived from suitable textbooks In specific embodiments of the present invention said gaseous compound may be or have the form of an immiscible fluid. Further examples of alternative immiscible fluids to be used here include ferrofluids.

A peristaltic actuation may be implemented, for example, by providing flexible connections or tubings in a device, or by setting up the device with one or more flexible or moveable sides or septa, allowing for a peristaltic movement. The peristaltic activity may be performed by any suitable means, e.g. a rolling device or actuator on said flexible tubings or septa, by a combination of two such tubings or septa in a contradirectional fashion, by the application of liquid mediated pressure on said tubings or septa, e.g. the provision of water filled tubing in direct contact with said tubings or septa according to the present invention, and/or by the use of a peristaltic pump. Further details and variations of this technique would be known to the person skilled in the art or could be derived from suitable textbooks.

A piston and actuated membrane driving may be implemented, for example, by the employment of at least one moveable surface of a device, or of a specific region of said device, e.g. of the reaction zone or reaction chamber, or of a mixing or repository zone as mentioned herein. This moveable surface may accordingly be in the form of a flexible membrane and be used as a piston decreasing the volume within said device, or within said zone of said device, leading to a transportation of neighboring material outside said zone or region. The moveable surface or piston structure may be combined with suitable expansion reservoirs or zones, allowing a movement of fluids inside the device. Further details and variations of this technique would be known to the person skilled in the art or could be derived from suitable textbooks.

A vectored vacuum may be implemented, for example, by the use of suitable vectored vacuum pumps sucking air or liquid into a specific zone of the device according to the present invention, e.g. into an expansion reservoir or target reservoir, or into a specific direction, leading to a movement of reagent solution and/or immiscible intermediate fluid units or plugs in the device. For instance, a compressed air and vacuum may be used to move membranes which are in contact with the fluid inside. Further details and variations of this technique would be known to the person skilled in the art or could be derived from suitable textbooks.

According to specific embodiments of the present invention the direction of the displacement activity, the flow rate and the velocity may be adjusted or changed according to suitability. In particular, the device may be equipped with two or more of the above mentioned movement or driving modules at different positions within said device allowing for (i) a fine-tuning of the displacement and (ii) a reversal of the direction of the displacement.

In another preferred embodiment of the present invention said separating step (d) is to be performed by allowing the above mentioned reagent solution, or reagent solutions, or any other solution, e.g. a washing buffer, and said immiscible intermediate fluid to separate by gravitational separation. The term "gravitational separation" as used herein refers to the employment of current, unenhanced gravitation over a certain period of time on a mixture of a reagent solution, or reagent solutions, or any other solution, e.g. a washing buffer, with said immiscible intermediate fluid. The gravitation leads, due to the nature of the intermediate fluid, in particular its immiscibility properties, to the generation of two or more phases within a specific zone, chamber or vessel. In the case of one reagent solution and one immiscible intermediate fluid the application of gravitation may produce a two phase liquid or fluid, one phase being the reagent solution and the other phase being the immiscible intermediate fluid. In case aqueous reagent solutions are employed the upper phase may be the reagent solution phase, while the lower phase may be the immiscible intermediate fluid

In preferred embodiments the gravitational separation may be enhanced by avoiding disturbances after a filling of a suitable zone, vessel or receptacle. This can, for instance, be implemented by a removal of said zone, vessel or receptacle from any inlet structure, e.g. by a rotator or a conveyer structure. By mechanically separating different reagent solution and immiscible intermediate fluid combinations or mixtures for other such reagent solution and immiscible intermediate fluid combinations an easy distinction between different reagent solutions can be implemented. Correspondingly removed zones, vessels or receptacles may subsequently be rejoined with the device, e.g. at introduction ports or via inlet structures, allowing for an effective reuse of the separated fluids.

In case of a gravitational separation of more than two different solutions/fluids, more than two outlet structures, e.g. located near to the corresponding phases, may be provided or used, allowing for an effective reuse of the separated fluids.

Difficultly separable fluids may additionally be separated by an enhanced gravitational separation, such as the use of centrifugal forces, a heating or cooling of the fluids, shaking movements or any other suitable activity.

In a further specific embodiment of the present invention said separating step (d) is to be performed by redirecting and/or distributing the above mentioned reagent solution, or reagent solutions, or any other solution, e.g. a washing buffer, or said immiscible intermediate fluid. The term "redirecting and/or distributing" as used herein refers to a deflection of fluid units as mentioned above into different directions, e.g. branching tubes or zones of the device and corresponding distribution of such units, e.g. within a network of such tubes or channels of a device. This activity may be implemented by the determination of a transition from an immiscible intermediate fluid to a reagent solution or another solution at a specific measurement point in the device. Subsequently, after having detected, for example, an immiscible intermediate fluid, the corresponding fluid unit may be moved or deflected towards or into a branching tubing, channel or receptacle. Similarly, after having detected, for example, a reagent solution, the corresponding fluid unit may be moved or deflected into a different direction, e.g. towards or into a branching tubing, channel or receptacle. Similar fluid units in said branching tubing, channel or receptacles may be rejoined or may be kept separate and be further transported or channeled back to the reaction zone of the device according to the present invention.

The transition between fluid units as described herein above may be detected by differences in color, viscosity, pH, electrical properties or other suitable parameters. Sensors or determination modules may be added to the device, e.g. in the vicinity of branching tubes.

In another aspect the present invention relates to a device for performing a molecular reaction, comprising: (a) a reaction zone which comprises a substrate, on which chemically or biochemically recognizable entities are immobilized; (b) reservoirs and liquid connections allowing a reagent solution, an immiscible intermediate fluid and optionally a washing buffer to pass through the reaction zone, wherein said immiscible intermediate fluid is capable of displacing said reagent solution from said reaction zone; and (c) a collection and regeneration zone wherein the immiscible intermediate fluid and the reagent solution are separable by gravitational separation, allowing for a reuse of the immiscible intermediate fluid and the reagent solution by channeling back the separated fluids to the reaction zone.

The device may preferably comprise a reaction zone as defined herein above, comprising chemically or biochemically recognizable entities as described herein above, being immobilized according to techniques described herein.

Said device may further comprise a reservoir, e.g. an expansion zone or vessel or an introduction vessel or zone allowing a movement of fluid units in the device, e.g. by one of the above mentioned transport techniques. A reservoir as mentioned here may have any suitable form or size and may comprise at least one inlet and one outlet, or more than one inlet and one outlet, or more than one outlet and one inlet. The reservoir may be open or be closed with the device. There may be one or more reservoirs present in the device, e.g. placed at different positions allowing for a movement of fluids etc.

Liquid connections as used herein may be of any size, diameter or form suitable. For instance, a liquid connection may be a capillary tube. Alternatively it may have a bigger size. Liquid connections do not necessarily have to be filled with liquid permanently, but can also be loaded with gaseous content, e.g. if a pneumatic displacement approach is to be performed.

A collection and regeneration zone as described herein above may be present. Such a zone is preferably provided in the form of a collection and regeneration container, vessel, chamber or receptacle. Said collection and regeneration zone may be suitable for accommodating one or more fluid units coming from a liquid connection, or a different zone of a device according to the present invention, e.g. driven by movement technique as described herein above. The collection and regeneration zone according to this aspect of the present invention is designed to allow a gravitational separation of fluid units of one or more reagent solution(s) and the immiscible intermediate fluid as described herein. Accordingly, in specific embodiments, said collection and regeneration zone may be designed to avoid disturbances after filling, e.g. by the provision of a rotational mechanism removing said zone from the device or from the inlet, by the provision of a removing mechanism for inlet connections, e.g. toward a further collection and regeneration zone, and/or by the provision of a stopping or pausing mechanism halting the filling of the collection and regeneration zone until a gravitational separation and/or reusing step has taken place, e.g. halting the filling process for 10 sec, 30 sec, 1 min, 2 min, 5 min, 10 min, 20 min etc.

The collection and regeneration zone may alternatively or additionally be equipped with elements allowing an enhanced separation as described herein above, e.g. an element allowing for the application of centrifugal forces, a cooling or heating element, a shaking or mixing module etc. These additional modules may be switched on in dependence of the separation status of the liquid in the collection and regeneration zone.

In a further preferred embodiment of the present invention said collection and regeneration zone comprises one inlet connected with the reaction zone as mentioned herein above and two outlets connected with a reservoir for the reagent solution and a reservoir for the immiscible intermediate fluid, respectively. The term "inlet connected with the reaction zone" as used herein refers to a direct or indirect liquid connection between the reaction zone as mentioned herein above, and the collection and regeneration zone. This connection may be implemented by the presence of a tubing, a channel, a capillary channel, a bridge or any other suitable connecting element. It may also be a direct connection between the reaction zone and the collection and regeneration zone, which can be shut, e.g. by a gate or lock. The term "outlet connected with a reservoir for the reagent solution" as used herein refers to a direct or indirect liquid connection between the collection and regeneration zone as mentioned herein above, and an additional reservoir, which only comprises reagent solution, preferably a reagent solution of one type or defined ingredients. This connection may be implemented by the presence of a tubing, a channel, a capillary channel, a bridge or any other suitable connecting element. A collection and regeneration zone may, in certain embodiments, be connected with more than one reservoir for a reagent solution, or may be connected with one reservoir for each type of reagent solution used in device. The term "outlet connected with a reservoir for the immiscible intermediate fluid" as used herein refers to a direct or indirect liquid connection between the collection and regeneration zone as mentioned herein above, and an additional reservoir, which only comprises an immiscible intermediate fluid according to the present invention. This connection may be implemented by the presence of a tubing, a channel, a capillary channel, a bridge or any other suitable connecting element.

Reservoirs for a reagent solution and for the immiscible intermediate fluid may be connected with the reaction zone of the device, allowing a rechanneling and an reuse of the respective fluids, or with any other zone or region of the device, e.g. with an introduction zone as mentioned herein above.

In another embodiment of the present invention said reservoir or reservoirs for a reagent solution and for the immiscible intermediate fluid may be removable from the device, e.g. allowing storage in an external place such a refrigerator or a cooling facility. Such removability may be implemented by the presence of gating or locking elements in the inlet and/or outlet sections. The possibility of removing the reservoirs form a device according to the present invention further increases the reusability of reaction solutions, e.g. comprising valuable enzymes or other ingredients, by providing for a further employment in a different device or on different point in time, e.g. after a storage period. Alternatively, removable reservoirs may also be employed for storage of reaction solutions, e.g. comprising valuable enzymes or other ingredients, before use. In specific embodiments the locking may also be of permanent nature. Inlet structures as described herein above may be designed in a way allowing an effective introduction of fluid units, e.g. by being position at the top of a collection and regeneration zone. Outlet structures may accordingly be designed in a way allowing the effective withdrawal of reagent solutions and immiscible intermediate fluids. For example, the outlets may be positions in the lower and upper half, third or quarter of a collection and regeneration zone. The inlets may alternatively be moveable relocatable according to the amount of liquid in the collection and regeneration zone. Alternatively, the collection and regeneration zone may comprise more than one outlet structure, which is preferably closable and may be used in accordance with the amount of liquid and the position of reagent solution and immiscible intermediate fluid phases.

The inlet and outlet structures may further be equipped with suitable driving elements, e.g. pumps, or with suitable blocking entities, such as gates, locks, or with branching tubules etc.

In a further specific embodiment of the present invention the collection and regeneration zone may be directly connected with said reaction zone or an introduction zone of the device, e.g. allowing a direct recycling of the reagent solution(s) and the immiscible intermediate fluid, e.g. via direct liquid connections.

In further embodiments of the present invention a washing buffer may also be separated from the immiscible intermediate fluid and/or a reagent solution provided said washing buffer is not miscible with said reagent. In case a washing buffer is used in a device, a collection and regeneration zone may be provided allowing for a gravitational separation of said washing buffer and the immiscible intermediate fluid as described herein. A corresponding collection and regeneration zone may accordingly be equipped with an inlet connected with a reaction zone or other zones of the device, and an outlet for the used washing buffer, leading to a waste module and an outlet for the immiscible intermediate fluid leading to a reservoir or directly back to other device zones, e.g. the reaction zone, as described herein. Alternatively, said washing buffer may also be reused and accordingly be transferred to a reservoir and/or directly back to other zones of the device, in particular the reaction zone as described herein above. For a reuse of the washing buffer the device may provide a filtering or sterilization module.

In a further aspect the present invention relates to an alternative device for performing a molecular reaction, comprising: (a) a reaction zone which comprises a substrate, on which chemically or biochemically recognizable entities are immobilized; (b) reservoirs and liquid connections allowing a reagent solution, an immiscible intermediate fluid and optionally a washing buffer to pass through the reaction zone, wherein said immiscible intermediate fluid is capable of displacing said reagent solution from said reaction zone; and (c) a redirection and distribution module, wherein the transition from the immiscible intermediate fluid to the reagent solution(s) is detected and said fluid and solution(s) are separated, allowing for a reuse of the immiscible intermediate fluid and the reagent solution by channeling back the separated fluids to the reaction zone. Reactions zones (a) and reservoirs and liquid connections (b) have already been described herein above.

A "redirection and distribution module" as mentioned herein refers to a technical entity deflecting or redirecting fluid units as mentioned above into different directions, e.g. into branching tubes or zones of the device, e.g. connected to a reaction zone of a device as described herein, wherein a reaction solution unit is deflected in one specific direction, whereas an immiscible intermediate fluid unit is deflected into another specific direction, which is not identical to the direction in which the reaction solution unit was deflected. Similarly, a differentially deflection of different types of reagent solution is possible. A corresponding deflection may be performed by a movement or driving technique as mentioned herein above, e.g. a pneumatic driving, a piston driving, or the application of vectored vacuum, the use of one or more valves. A prerequisite for an effective deflecting is the detection of a transition between non-identical fluid units. The transition between fluid units may be detected as described herein above, e.g. by determining differences in color, viscosity, pH, electrical properties or other suitable parameters with suitable sensors, cameras or other technical entities known to the person skilled in the art. Such technical entities are preferably connected with said deflecting module, e.g. via microelectronic circuits or a computerized assessment module, allowing, for example, an automated or semi-automated distribution of fluid units in device. Further sensors or determination modules may also be provided in other zones of the device, e.g. in the vicinity of branching tubes.

In a specific embodiment of the present invention said redirection and distribution module may comprises one inlet connected with the reaction zone and two outlets connected with a reservoir for the reagent solution and a reservoir for the immiscible intermediate fluid, respectively. The term "inlet connected with the reaction zone" as used herein refers to a direct or indirect liquid connection between the reaction zone as mentioned herein above, and the redirection and distribution module. This connection may be implemented by the presence of a tubing, a channel, a capillary channel, a bridge or any other suitable connecting element. It may also be a direct connection between the reaction zone and the redirection and distribution module, which can be shut, e.g. by a gate or lock. The term "outlet connected with a reservoir for the reagent solution" as used herein refers to a direct or indirect liquid connection between the redirection and distribution module as mentioned herein above, and an additional reservoir, which only comprises reagent solution, preferably a reagent solution of one type or defined ingredients. This connection may be implemented by the presence of a tubing, a channel, a capillary channel, a bridge or any other suitable connecting element. A redirection and distribution module may, in certain embodiments, be connected with more than one reservoir for a reagent solution, or may be connected with one reservoir for each type of reagent solution used in device. The term "outlet connected with a reservoir for the immiscible intermediate fluid" as used herein refers to a direct or indirect liquid connection between the redirection and distribution module as mentioned herein above, and an additional reservoir, which only comprises an immiscible intermediate fluid according to the present invention. This connection may be implemented by the presence of a tubing, a channel, a capillary channel, a bridge or any other suitable connecting element. Reservoirs for a reagent solution and for the immiscible intermediate fluid may be connected with the reaction zone of the device, allowing a rechanneling and an reuse of the respective fluids, or with any other zone or region of the device, e.g. with an introduction zone as mentioned herein above. In another embodiment of the present invention said reservoir or reservoirs for a reagent solution and for the immiscible intermediate fluid may be removable from the device as defined herein above.

In a further specific embodiment of the present invention the redirection and distribution module may be directly connected with a reaction zone or an introduction zone of the device, e.g. allowing a direct recycling of the reagent solution(s) and the immiscible intermediate fluid, e.g. via direct liquid connections.

In further embodiments of the present invention a washing buffer may also be separated from the immiscible intermediate fluid and/or a reagent solution provided said washing buffer is not miscible with said reagent. In case a washing buffer is used in a device, a redirection and distribution module may be provided allowing for a redirection of said washing buffer and the immiscible intermediate fluid as described herein. A corresponding redirection and distribution module may accordingly be equipped with an inlet connected with a reaction zone or other zones of the device, and an outlet for the used washing buffer, leading to a waste module and an outlet for the immiscible intermediate fluid leading to a reservoir or directly back to other device zones, e.g. the reaction zone, as described herein. Alternatively, said washing buffer may also be reused and accordingly be transferred to a reservoir and/or directly back to other zones of the device, in particular the reaction zone as described herein above. For a reuse of the washing buffer the device may provide a filtering or sterilization module.

In further embodiments of the present invention a regeneration of ingredients in a reagent solution may be carried out after a repeated used. Furthermore, the quality of reagents in said reagent solution may be determined, e.g. in situ, or in external determination sections of the device.

In a further preferred embodiment of the present invention said device as mentioned herein above, or mentioned in the context of the method of the present invention is a microfluidic device. The term "micro fluidic device" as used herein refers to a device allowing the precise control and manipulation of fluids that are constrained to small, preferably sub-millimeter scales. Typically, a microfluidic device implements small volumes, e.g. in the range of nl, pl or fl, and/or it may implement an small overall size. Furthermore, a microfluidic device according to the present invention may consume a lower amount of energy. In a microfluidic device effects such as laminar flow, specific surface tensions, electrowetting, fast thermal relaxation, the presence of electrical surface charges and diffusion effects may be implemented and/or used for the performance of a method according to the present invention. In certain embodiments, a microfluidic device may have connections with external sources or external elements, e.g. the separation or reservoirs or vessels for reuse purposes may be possible. Furthermore, the microfluidic device may comprise an electronic or computer interface allowing the control and manipulation of activities in the device, and/or the detection or determination of reaction outcomes or products.

In another specific embodiment of the present invention said microfluidic device may be an integrated microfluidic cartridge. The term "integrated microfluidic cartridge" as used herein refers to the compactation and resizing of a microfluidic, e.g. comprising all necessary connections, zones and, optionally, also necessary ingredients with a chamber or container like form. The cartridge may, for example, be entirely closed, or partially closed allowing the introduction of samples, ingredients etc. via resealable inlets. The cartridge may further be equipped with alignment structures for scanning or detection devices, e.g. recognition o portions for a scanner, a bar code or matrix code indicating the provided ingredients, the manufacture date etc., or an interface to a detection unit allowing the electronic or optical determination of reaction outcomes as mentioned herein above. In further preferred embodiments said cartridges may comprise on board reagent solution(s) and/or immiscible intermediate fluids, e.g. for the purpose of single use in clinical applications, allowing to avoid cross contamination.

In a further particularly preferred embodiment of the present said molecular reaction as described herein above, in the context of methods and devices according to the present invention, is a sequencing reaction or a nucleic acid synthesis reaction.

The term "sequencing reaction" as used herein refers to one or more reactions used in methods for sequence determination Such methods for sequence determination are known to the person skilled in the art. These methods include next generation sequencing methods or high throughput sequencing methods, for example, a pyrosequencing based on the technique provided by Roche for the 454 Genome Sequencer. This method amplifies DNA inside water droplets in an oil solution with each droplet containing a single DNA template attached to a single primer-coated bead that then forms a clonal colony. Pyrosequencing uses luciferase to generate light for detection of the individual nucleotides added to the nascent DNA, and the combined data are used to generate sequence read-outs. Another example is the methodology of Illumina or Solexa sequencing, which is based on reversible dye-terminators. DNA molecules are typically attached to primers on a slide and amplified so that local clonal colonies are formed. Subsequently one type of nucleotide at a time may be added, and non-incorporated nucleotides are washed away. Subsequently, images of the fluorescently labeled nucleotides may be taken and the dye is chemically removed from the DNA, allowing a next cycle. A further example is the Applied Biosystems' SOLiD technology, which employs sequencing by ligation. This method is based on the use of a pool of all possible oligonucleotides of a fixed length, which are labeled according to the sequenced position. Such oligonucleotides are annealed and ligated. Subsequently, the preferential ligation by DNA ligase for matching sequences typically results in a signal informative of the nucleotide at that position. Since the DNA is typically amplified by emulsion PCR, the resulting bead, each containing only copies of the same DNA molecule, can be deposited on a glass slide resulting in sequences of quantities and lengths comparable to Illumina sequencing. Yet another example is Helicos' Heliscope technology, wherein fragments are captured by polyT oligomers tethered to an array. At each sequencing cycle, polymerase and single fluorescently labeled nucleotides are added and the array is imaged. The fluorescent tag is subsequently removed and the cylce is repeated. Further examples of sequencing techniques encompassed within the present invention are microfluidic Sanger sequencing, and microchip-based sequencing methods. The present invention also envisages further developments of these techniques, e.g. further improvements of the accuracy of the sequence determination, or the time needed for the determination of the genomic sequence of an organism etc. These sequencing methods may be adapted within the presently provided methodology, allowing for a highly increased reading length and reduced costs due to the possibility of increasing the amount of enzymes and/or oligomers and/or nucleotides during the performance of the reactions. Further variants and details of the methods would be known to the person skilled in the art, and can be derived from suitable publications e.g. Metzger, 2010, Nature Reviews, Genetics, Vol 11.

The term "nucleic acid synthesis reaction" as used herein refers to methods of direct production of oligonucleotide sequences on a substrate, e.g. in the form of an array. A typical method may, for example, involve a photolithographic synthesis on a silica substrate where light and light-sensitive masking agents are utilized to generate a sequence one nucleotide at a time. Each applicable probe is selectively unmasked prior to the provision of a solution of a single nucleotide, then a masking reaction takes place and the next set of probes are unmasked in preparation for a different nucleotide exposure. After several repetitions, the sequences of the envisaged probe become fully constructed. Accordingly constructed oligonucleotides may be longer (e.g. 60-mers) or shorter (e.g. 25-mers) depending on the desired purpose. Further variants and details of the methods would be known to the person skilled in the art, and can be derived from suitable publications e.g. from Pease et al., 1994, PNAS 91: 5022-5026.

In another aspect the present invention relates to the use of an immiscible intermediate fluid as defined herein for displacing a reagent solution, or alternatively a washing solution or buffer, present in a reaction zone in a microfluidic device as defined herein from said reaction zone substrate. Preferably, a displacement of said reagent solution or washing solution or buffer, may be a complete or substantially complete displacement, e.g. leading to a 99%, 99.9%, 99.99% or 100% removal of the reagent solution or of any other solution from the reaction zone substrate.

In a preferred embodiment of the present invention said immiscible intermediate fluid as defined herein may be used for the displacing of a reagent solution, or alternatively a washing solution or buffer, from a reaction zone, wherein the reaction zone comprises a substrate with chemically or biochemically recognizable entities.

In an even more preferred embodiment of the present invention said immiscible intermediate fluid as defined herein may be used for rendering said chemically or biochemically recognizable units active or reactive by displacing a reagent solution, or alternatively a washing solution or buffer, from said reaction zone substrate. The term "rendering active or reactive" as used herein refers to a removal of any binding or wetting entity on chemically or biochemically entities in or on said substrate. The substrate may accordingly be cleaned from waste products, binding entities, buffer components, enzymatic substrates etc.

In another particularly preferred embodiment of the present invention, said chemically or biochemically recognizable entities are nucleic acids, peptides, polypeptides, carbohydrates, or modified versions thereof, or an array of nucleic acids, peptides, polypeptides, carbohydrates or modified versions thereof or any combination thereof.

The term "nucleic acid" as used herein refers to any nucleic acid known to the person skilled in the art, preferably to DNA, RNA, PNA, CNA, HNA, LNA or ANA. The DNA may be in the form of, e.g. A-DNA, B-DNA or Z-DNA. The RNA may be in the form of, e.g. p-RNA, i.e. pyranosysl-RNA or structurally modified forms like hairpin RNA or a stem-loop RNA. The term "PNA" relates to a peptide nucleic acid, i.e. an artificially synthesized polymer similar to DNA or RNA which is used in biological research and medical treatments, but which is not known to occur naturally. The PNA backbone is typically composed of repeating N-(2-aminoethyl)-glycine units linked by peptide bonds. The various purine and pyrimidine bases are linked to the backbone by methylene carbonyl bonds. PNAs are generally depicted like peptides, with the N-terminus at the first (left) position and the C-terminus at the right. The term "CNA" relates to an aminocyclohexylethane acid nucleic acid. Furthermore, the term relates to a cyclopentane nucleic acid, i.e. a nucleic acid molecule comprising for example 2'-deoxycarbaguanosine.

The term "HNA" relates to hexitol nucleic acids, i.e. DNA analogues which are built up from standard nucleobases and a phosphorylated 1, 5-anhydrohexitol backbone. The term "LNA" relates to locked nucleic acids. Typically, a locked nucleic acid is a modified and thus inaccessible RNA nucleotide. The ribose moiety of an LNA nucleotide may be modified with an extra bridge connecting the 2' and 4' carbons. Such a bridge locks the ribose in a 3'-endo structural conformation. The locked ribose conformation enhances base stacking and backbone pre-organization. This may significantly increase the thermal stability, i.e. melting temperature of the oligonucleotide. The term "ANA" relates to arabinoic nucleic acids or derivatives thereof. A preferred ANA derivative in the context of the present invention is a 2'-deoxy-2'-fluoro-beta-D-arabinonucleoside (2'F-ANA). In a further embodiment nucleic acid molecules may comprise a combination of any one of DNA, RNA, PNA, CNA, HNA, LNA and ANA. In a further embodiment the nucleic acid molecules as defined herein above may be in the form of short oligonucleotides, long oligonucleotides or polynucleotides.

The term "peptide" in the context of the present invention refers to any type of amino acid sequence comprising more than 1 amino acids, in particular short sequences of up to 40 or 50 amino acids length. The term, in certain embodiments, also refers protein structures or functional derivatives thereof. Furthermore, the peptide may be combined with further chemical moieties or functionalities.

The terms "polypeptide" as used herein refers medium or long amino acid sequences, preferably comprising 40 or 50 amino acids. The term, in certain embodiments, also refers protein structures or functional derivatives thereof. Furthermore, the polypeptide may be combined with further chemical moieties or functionalities.

The term "carbohydrate" as used herein refers to an organic compound with the empirical formula C*ₘ*(H₂O)*ₙ*; that is, consists only of carbon, hydrogen, and oxygen, with a hydrogen:oxygen atom ratio of 2:1. The group comprises monosaccharides, disaccharides, oligosaccharides, and polysaccharides. Reference is preferably made to sugars, i.e. to monosaccharides and disaccharides.

A "modification" of the polypeptide, protein or peptide according to the present invention may be an acetylation, pegylation, hesylation, formylation, phosphorylation, amidation, derivatization by known protecting/blocking groups, specific chemical cleavage, proteolytic cleavage, a linkage to a cellular ligand or other protein or hapylation, i.e. a fusion with a glycine-rich homo-amino-acid polymer (HAP), etc. Such modifications may be carried out by suitable techniques known to the person skilled in the art. Additionally, the polypeptide, peptide or variant may contain one or more non-classical amino acids.

An "array" as used herein refers to an ordered array presented for interaction between capture molecules in the array and potential interactors in the surrounding environment, e.g. a medium as described herein above. An array may include any two- or three-dimensional arrangement of addressable regions, preferably a two-dimensional arrangement. The array may be comprised of nucleic acids, e.g. oligonucleotides, peptide/polypeptide entities, carbohydrates or a mixture or combination of these.

In a preferred embodiment of the present invention said immiscible intermediate fluid comprises fluorinated hydrocarbons. In a further preferred embodiment of the present invention said immiscible intermediate fluid consists of fluorinated hydrocarbons. In another preferred embodiment of the present invention said immiscible intermediate fluid comprises per fluorinated hydrocarbons. In another preferred embodiment of the present invention said immiscible intermediate fluid consists of per fluorinated hydrocarbons. In another preferred embodiment of the present invention said immiscible intermediate fluid comprises fluorinated silicones. In another preferred embodiment of the present invention said immiscible intermediate fluid consists of fluorinated silicones. In another preferred embodiment of the present invention said immiscible intermediate fluid comprises per fluorinated silicones. In another preferred embodiment of the present invention said immiscible intermediate fluid consists of per fluorinated silicones. In another preferred embodiment of the present invention said immiscible intermediate fluid comprises fluorinated saturated hydrocarbons. In another preferred embodiment of the present invention said immiscible intermediate fluid consists of fluorinated saturated hydrocarbons. In another preferred embodiment of the present invention said immiscible intermediate fluid comprises per fluorinated saturated hydrocarbons. In another preferred embodiment of the present invention said immiscible intermediate fluid consists of per fluorinated saturated hydrocarbons.

In certain embodiments of the present invention said immiscible intermediate fluid comprises or consists of a combination of the above mentioned hydrocarbons.

The term "hydrocarbon" as used herein refers to a molecule consisting primarily of hydrogen and carbon. The group comprises hydrocarbyls, aromatic hydrocarbons (arenes), alkanes, alkenes, cycloalkanes and alkyne-based compounds. Preferably, the term relates to alkanes such as butane, pentane, hexane, heptane, octane, nonane, decane etc. or to alkene such as pentene, hexene, heptene, octene, nonene, decene etc. In certain embodiments of the present invention the group of hydrocarbons may also comprise hetero atoms, in particular nitrogen, or the group of hydrocarbons may additionally comprise functional groups, in particular an amine group.

The term "silicone" as used herein refers to polymerized siloxanes or polysiloxanes, i.e. mixed inorganic-organic polymers with the general formula [R₂SiO]ₙ, where R is an organic group such as methyl, ethyl, or phenyl.

A "fluorinated hydrocarbon" or a "fluorinated silicone" according to the present invention is a hydrocarbon or a silicon where one, or more, but not all hydrogens rare substituted by fluorine atoms. It is perferred to have or use hydrocarbons and/or silicones with a high degree of fluorination, providing a low solubility of hydrophilic and hycrophobic substances.

A "per fluorinated hydrocarbon" or a "per fluorinated silicone" according to the present invention is a hydrocarbon or a silicon molecule where all hydrogen atoms are substituted by fluorine atoms.

In a further preferred embodiment of the present invention said immiscible intermediate fluid has a viscosity of less than about 0.1 Pa.s, a density of more than about 1100 kg/l, a boiling point higher than about 50°C and a solubility of water of less than about 1%.

In certain embodiments of the present invention, said immiscible intermediate fluid may, for example have a viscosity of about 0.09 Pa.s, 0.08 Pa.s, 0.07 Pa.s, 0.06 Pa.s, 0.05 Pa.s, 0.04 Pa.s, 0.03 Pa.s, 0.02 Pa.s, 0.01 Pa.s, etc. and a density of more than about 1100 kg/l, a boiling point higher than about 50°C and a solubility of water of less than about 1%.

In further embodiments of the present invention, said immiscible intermediate fluid may, for example, have a density of about 1200 kg/l, 1300 kg/l, 1400 kg/l, 1500 kg/l, 1750 kg/l, 2000 kg/l, 2500 kg/l etc. and a viscosity of less than about 0.1 Pa.s, a boiling point higher than about 50°C and a solubility of water of less than about 1%.

In further embodiments of the present invention, said immiscible intermediate fluid may, for example, have a boiling point of about 60°C, 70°C, 80°C, 90°C, 100°C, 110°C, 120°C, 130°C, 140°C, 150°C or higher, and a viscosity of less than about 0.1 Pa.s, a density of more than about 1100 kg/l, and solubility of water of less than about 1%.

In further embodiments of the present invention, said immiscible intermediate fluid may, for example, have a solubility of water of about 0.9%, 0.8%, 0.7%, 0.6%, 0.5%, 0.4%, 0.3%, 0.2%, 0.1% or less and a viscosity of less than about 0.1 Pa.s, a density of more than about 1100 kg/l, and a boiling point higher than about 50°C.

In further embodiments of the present invention, said immiscible intermediate fluid may, for example, have a viscosity of about 0.09 Pa.s, 0.08 Pa.s, 0.07 Pa.s, 0.06 Pa.s, 0.05 Pa.s, 0.04 Pa.s, 0.03 Pa.s, 0.02 Pa.s, or 0.01 Pa.s, etc; and a density of about 1200 kg/l, 1300 kg/l, 1400 kg/l, 1500 kg/l, 1750 kg/l, 2000 kg/l, or 2500 kg/l etc.; and a boiling point of about 60°C, 70°C, 80°C, 90°C, 100°C, 110°C, 120°C, 130°C, 140°C, 150°C or higher; and a solubility of water of about 0.9%, 0.8%, 0.7%, 0.6%, 0.5%, 0.4%, 0.3%, 0.2%, 0.1% or less.

The above mentioned parameter(s) and/or the immiscibility of fluids may be tested and determined according to suitable assays and procedures known to the skilled person. The present invention accordingly envisages fluids which fall within the above provided group parameters and being immiscible, e.g. as determined by a suitable test or assay.

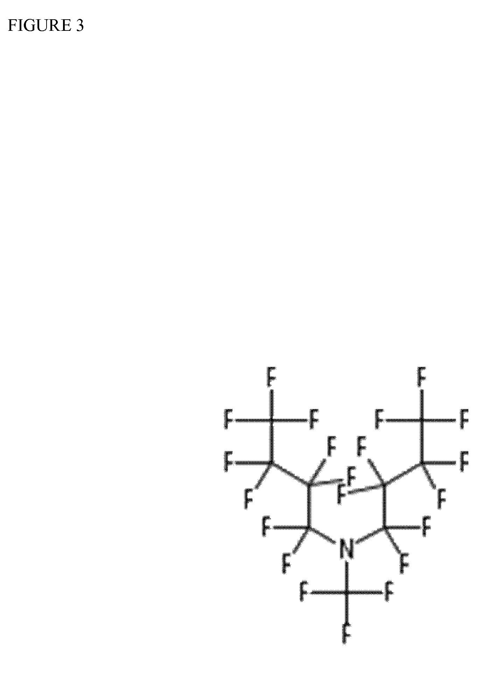

In another preferred embodiment of the present invention said immiscible intermediate fluid is Fluorinert Electronic Liquid FC-40. The term refers, in particular, to a compound as depicted in Fig. 3, and further having the following properties: (i) appearance: clear, colorless, (ii) average molecular weight: 650; (iii) boiling point (1 atm): 165°C; (iv) pour point: -57°C; (v) calculated critical temperature: 543 K; (vi) estimated critical pressure: 1.18 × 10⁶ pascals; (vii) vapor pressure: 287 pascals; (vii) latent heat of vaporization (at normal boiling point) : 69 J/g; (vii) liquid density: 1855 kg/m³; (viii) kinematic viscosity: 2.2 centistokes; (ix) absolute viscosity: 4.1 centipoise; (x) liquid specific heat: 1100 J kg⁻¹ °c⁻¹; (xi) liquid thermal conductivity: 0.065 W m⁻¹ °c⁻¹; (xii) coefficient of expansion: 0.0012°c⁻¹; (xiii) refractive index: 1.290; (xiii) water solubility <7 ppmw; (xiv) solubility in water: <5; ppmw; (xv) dielectric strength: 46 kV, 0.1" gap; (xvi) dielectric constant: 1.9; and (xvii) electrical resistivity (ASTM D-257): 4.0 ×1015 ohm cm.

In further specific embodiments of the present invention FC-40 may be combined with one or more of other immiscible intermediate fluids, e.g. one or more of the herein above described immiscible intermediate fluids.

In yet another preferred embodiment the present invention relates to the use of a device as defined herein above for performing a sequence reaction or a nucleic acid synthesis reaction. A sequence reaction in the context of the present use claim is to be understood as a sequencing reaction as defined herein above, or as known to the person skilled in the art. A nucleic acid synthesis reaction in the context of the present use claim is to be understood as a nucleic acid synthesis reaction as defined herein above, or as known to the person skilled in the art. Further envisaged is the use of the device as defined herein above for molecular reactions as mentioned herein above, or suitable molecular reactions as known to the person skilled in the art.

The following example and figures are provided for illustrative purposes. It is thus understood that the example and figures are not to be construed as limiting. The skilled person in the art will clearly be able to envisage further modifications of the principles laid out herein.

### EXAMPLES

### Example 1 - Effect of FC-40 on hybridization signals

In order to test the effect of FC-40 on hybridization signals, a hybridization was performed for two different probes, i.e. PCR 12 and PCR5at a concentration of 1 nM.

PCR product 12 comprises of 114 base pairs and is part of the human chromosome with the accession No. NCBI36:12:111399702:111400602:1 as of 15 March 2011; the sequence is reported below (SEQ ID NO: 1), the area of specific binding to the capture probes is underlined:

AGTTACATTGCCACACAAGGCTGCCTGCAAAACACGGTGAATGACT
TTTGGCGGATGGTGTTCCAAGAAAACTCCCGAGTGATTGTCATGACAACGAAAG
AAGTGGAGAGAGGA (SEQ ID NO:1).

PCR product 5 comprises of 108 base pairs and is part of the human chromosome the accession No. NCBI36:5:127637813:127638713:1; as of 15 March 2011; the sequence is reported below (SEQ ID NO: 2); the area of specific binding to the capture probes is underlined:

ACCAGGTGGACATTTACAGGTAAACCCCCCCAGGGTGTTGACACAG
AGGAACTGGCAGTTATGCTGCTTTGTTTGACATTCATCAAGGTCTGAAATTAGAG
AGAAGCC (SEQ ID NO:2).

PCR products were synthesized and fluorescently labeled by Atto770 (Biolegio). Two types of synthetic oligonucleotides, both 76 nucleotides length (Biolegio), were used as capture probes for PCR12 and PCR5, respectively:

### Capture probe 12:

GGAGTTTTCTTGGAACACCATCCGCCAAAAGTCATTCAC
CGTGTTTTGCAGGCAGCCTT (SEQ ID NO:3).

### Capture probe 5:

CTTGATGAATGTCAAACAAAGCAGCATAACTGCCAGTTC
CTCTGTGTCAACACCCTGGGG (SEQ ID NO:4).

Oligonucleotides were dissolved in PBS containing betaine 1.5 M at a concentration of 6 µM, ink-jet printed in rows in a clean room facility on aminosilane glass slide (Genorama SA slides) and UV crosslinked; each spot was produced by 300 pL of solution.

Reference spots fluorescently labeled by Atto700 were dissolved in PBS containing betaine 1.5 M at concentration of 2 µM and printed in a parallel row.

Microfluidic structures with inlet, outlet and U shaped microchannel (width 1.2mm, total length 50 mm, height 100 µm) were built on the printed area, in order to perform flow over hybridization tests with PCR 12 and PCR 5. Hybridization solution is injected via the inlet by means of a syringe pump in the microchannel.

PCR 12 and PCR 5 were dissolved in hybridization buffer (3x SSC w 0.11% SDS) at a concentration of 1 nM, denatured for 10 minutes at 95°C, cooled down in ice and injected in the microfluidic structure by means of a syringe pump at 6 µl/min. The flow was maintained constant for 30 minutes.

The system was kept on a hot plate to ensure the temperature of 54°C inside the microchannel during the hybridization.

After 30 minutes of flow over hybridization at 54°C, the fluid was removed from the microchannel and the area was analyzed by confocal scanner to detect the binding of labeled PCR products.

In order to assess the inertness of FC-40 on the hybridization process, three parallel experimental approaches were carried out under the following conditions:
1) Flow over of FC-40 for 5 minutes, room temperature, at 6 µl/min + hybridization of PCR 12 and PCR5 as previously described;
2) FC-40 for 5 mins, 6 µl/min + washing buffer (1x SSC w 0.2% SDS) for 5 minutes, room temperature, at 6 µl/min + hybridization of PCR 12 and PCR5 as previously described; and
3) Hybridization of PCR 12 and PCR5 as previously described, without contact to FC-40

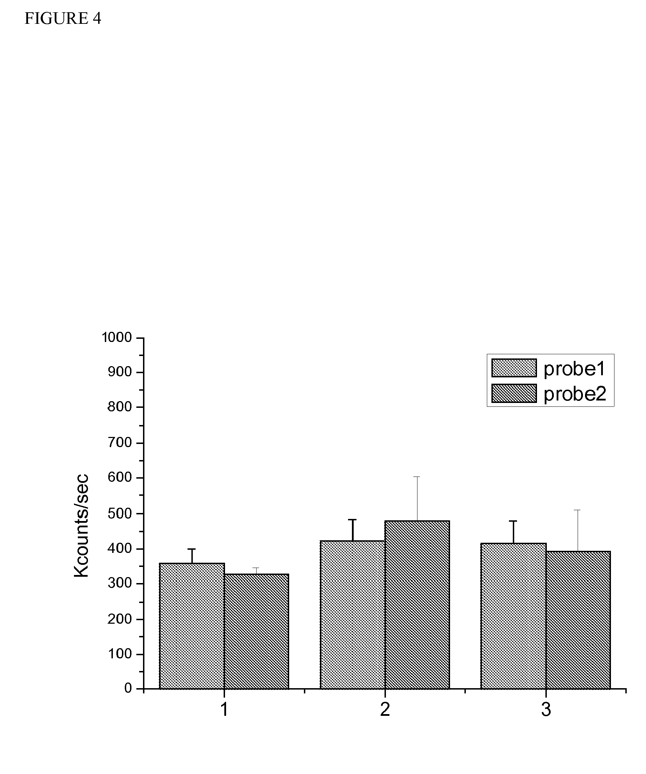

As can be derived from Fig. 4, sections 1, 2 and 3 (corresponding to experimental sections 1), 2) and 3), supra) the contact with FC-40 has no effect on the hybridization signal.

As can be derived from Fig. 4, sections 1, 2 and 3 (corresponding to experimental sections 1), 2) and 3), supra) the contact with FC-40 has no effect on the hybridization signal.

### Example 2 - Effect of FC-40 on the enzymatic end-repair PRC reaction

In order to test the effect of FC-40 on enzymatic end-repair PRC reaction a hybridization with Cp40am, a synthetic oligonucleotide with amino functionality, 58 nucleotides length (Biolegio) was carried out. The sequence of Cp40am is reported below (SEQ ID NO: 5):

Cp40am: AGTCCTCACCCAAGCGCACGTTTTCGATTAGCTGCCCA
AGTCTTCAATGCATCTTACC (SEQ ID NO: 5).

Cp40am was dissolved in PBS w betaine 1.5 M and printed as capture probe on Nexterion slides P (Schott) as recommended by the datasheet. A 16-well superstructure (Schott) was applied to the printed slide.

As40+4, which is a complementary probe with 4 overhanging nucleotides (GCTA-), was dissolved in hybridization buffer (3x SSC w 0.1% SDS) to 10 nM; 50 µl of hybridization solution were introduced in the wells. Hybridization was carried out at 50°C for 30 minutes.

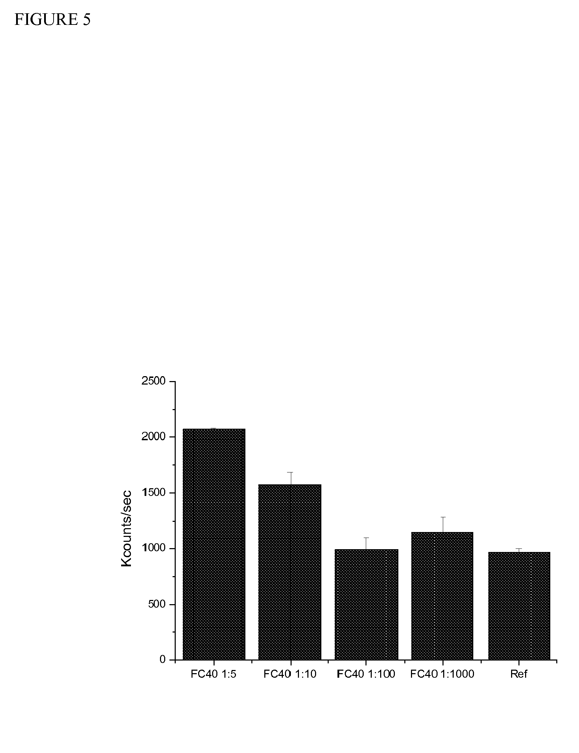

After washing in washing buffer (1x SSC w 0.2% SDS) end-repair reaction was performed with Klenow polymerase in NEB buffer w 0.1 % BSA with nucleotide mic dNTP-C*, where C nucleotide was labeled by Cy5; dNTP-C* mix was diluted to a concentration of 300 nM. This solution is considered as the reference solution (see Figure 5; "Ref").

In order to test the effect of FC-40 on the end-repair reaction increasing amount of FC-40 were added to the polymerase solution according to the following ratios
1:1000, 1:100, 1:10, 1:5

50 µl of each solution were applied to different wells and the end-repair reaction was carried out at 37°C for 30 minutes.

As can be derived from Fig. 5, 3^{rd} column, traces of FC-40 up to 1:100 (v/v) do not influence the activity of Klenow polymerase during the end-repair step. Surprisingly, a higher concentration of FC-40 (1^{st} and 2^{nd} column) appears to increase the yield of the polymerase reaction.

## Claims

1. Method for performing molecular reactions in a device comprising the following steps:
(a) introducing one or more reagent solution(s) and an immiscible intermediate fluid into the device, wherein the device comprises a substrate, on which chemically or biochemically recognizable entities are immobilized;
(b) performing molecular reactions between the immobilized chemically or biochemically recognizable entities and the reagent solution(s); or on the immobilized chemically or biochemically recognizable entities in the presence of the reagent solution(s);
(c) displacing the reagent solution(s) present on the substrate by the immiscible intermediate fluid;
(d) separating the immiscible intermediate fluid and the reagent solution(s), preferably in a collection and regeneration zone; and
(e) reusing the reagent solution(s) and/or immiscible intermediate fluid for one or more repetitions of steps (a) to (e).

2. The method of claim 1, additionally comprising the step of washing said substrate with a washing buffer, wherein said washing buffer is detached from said reagent solution(s) by the immiscible intermediate fluid.

3. The method of claim 1 or 2, additionally comprising the step of measuring the state or outcome of said molecular reaction, preferably by optical and/or electrical means.

4. The method of any one of claims 1 to 3, wherein said displacing step (c) is to be performed by pneumatic fluid driving, peristaltic actuation, piston and actuated membrane driving or the application of vectored vacuum.

5. The method of any one of claims 1 to 4, wherein said separating step (d) is to be performed by allowing said reagent solution(s) and said immiscible intermediate fluid to separate by gravitational separation.

6. Device for performing a molecular reaction, comprising:
(a) a reaction zone which comprises a substrate, on which chemically or biochemically recognizable entities are immobilized;
(b) reservoirs and liquid connections allowing a reagent solution, an immiscible intermediate fluid and optionally a washing buffer to pass through the reaction zone, wherein said immiscible intermediate fluid is capable of displacing said reagent solution from said reaction zone; and
(c) a collection and regeneration zone wherein the immiscible intermediate fluid and the reagent solution are separable by gravitational separation, allowing for a reuse of the immiscible intermediate fluid and the reagent solution by channeling back the separated fluids to the reaction zone, wherein said collection and regeneration zone optionally comprises one inlet connected with the reaction zone and two outlets connected with a reservoir for the reagent solution and a reservoir for the immiscible intermediate fluid, respectively.

7. Device for performing a molecular reaction, comprising:
(a) a reaction zone which comprises a substrate, on which chemically or biochemically recognizable entities are immobilized;
(b) reservoirs and liquid connections allowing a reagent solution, an immiscible intermediate fluid and optionally a washing buffer to pass through the reaction zone, wherein said immiscible intermediate fluid is capable of displacing said reagent solution from said reaction zone; and
(c) a redirection and distribution module, wherein the transition from the immiscible intermediate fluid to the reagent solution(s) is detected and said fluid and solution(s) are separated, allowing for a reuse of the immiscible intermediate fluid and the reagent solution by channeling back the separated fluids to the reaction zone, wherein said redirection and distribution module optionally comprises one inlet connected with the reaction zone and two outlets connected with a reservoir for the reagent solution and a reservoir for the immiscible intermediate fluid, respectively.

8. The method of any one of claims 1 to 5, or the device of claims 6 or 7, wherein said device is a microfluidic device, preferably an integrated micro fluidic cartridge.

9. The method of any one of claims 1 to 5 or 8, or the device of any one of claims 6 to 8, wherein said molecular reaction is a sequencing reaction or a nucleic acid synthesis reaction.

10. Use of an immiscible intermediate fluid for displacing a reagent solution present in a reaction zone in a micro fluidic device from said reaction zone substrate, wherein the reaction zone comprises a substrate with chemically or biochemically recognizable entities, and rendering said chemically of biochemically recognizable units (re-)active.

11. The method of any one of claims 1 to 5, 8 or 9, the device of any one of claims 6 to 9, or the use of claim 10, wherein said chemically or biochemically recognizable entities are nucleic acids, peptides, polypeptides, carbohydrates, modified versions thereof, an array of nucleic acids, peptides, polypeptides, carbohydrates or modified versions thereof or any combination thereof.

12. The method of any one of claims 1 to 5, 8 9, or 11, the device of any one of claims 6 to 9 or 11, or the use of claim 10 or 11, wherein said immiscible intermediate fluid comprises or consists of (per) fluorinated hydrocarbons and/or (per) fluorinated silicones, preferably comprises or consists of (per) fluorinated saturated hydrocarbons.

13. The method of any one of claims 1 to 5, 8, 9 or 11, the device of any one of claims 6 to 9 or 11, or the use of claim 10 or 11, wherein said immiscible intermediate fluid has a viscosity of less than about 0.1 Pa.s, a density of more than about 1100 kg/l, a boiling point higher than about 50°C and a solubility of water of less than about 1%.

14. The method of any one of claims 1 to 5, 8, 9, 11, 12 or 13, the device of any one of claims 6 to 9, 11, 12 or 13, or the use of any one of claims 10, 11, 12 or 13, wherein said immiscible intermediate fluid is Fluorinert Electronic Liquid FC-40.

15. Use of a device of any one of claims 6 to 9, 11, 12, 13 or 14 for performing a sequencing reaction or a nucleic acid synthesis reaction.


SEQUENCE LISTING

<110>  Koninklijke Philips Electronics N.V.

<120>  METHOD FOR PERFORMING MOLECULAR REACTIONS BY USING IMMISCIBLE
       INTERMEDIATE FLUIDS

<130>  PH016439 EP1

<160>  5

<170>  PatentIn version 3.5

<210>  1
<211>  114
<212>  DNA
<213>  Homo sapiens

<400>  1
agttacattg ccacacaagg ctgcctgcaa aacacggtga atgacttttg gcggatggtg      60

ttccaagaaa actcccgagt gattgtcatg acaacgaaag aagtggagag agga           114


<210>  2
<211>  108
<212>  DNA
<213>  Homo sapiens

<400>  2
accaggtgga catttacagg taaacccccc cagggtgttg acacagagga actggcagtt      60

atgctgcttt gtttgacatt catcaaggtc tgaaattaga gagaagcc                  108


<210>  3
<211>  59
<212>  DNA
<213>  Artificial Sequence

<220>
<223>  Capture probe

<400>  3
ggagttttct tggaacacca tccgccaaaa gtcattcacc gtgttttgca ggcagcctt       59


<210>  4
<211>  60
<212>  DNA
<213>  Artificial Sequence

<220>
<223>  Capture Probe

<400>  4
cttgatgaat gtcaaacaaa gcagcataac tgccagttcc tctgtgtcaa caccctgggg      60


<210>  5
<211>  58
<212>  DNA
<213>  Artificial Sequence

<220>
<223>  synthetic oligonucleotide

<400>  5
agtcctcacc caagcgcacg ttttcgatta gctgcccaag tcttcaatgc atcttacc        58